# EUROPEAN PATENT SPECIFICATION

(11) **EP 2 904 104 B1**
(45) Date of publication and mention of the grant of the patent: **15.08.2018**
(21) Application number: 13783139.2
(22) Date of filing: 02.10.2013
(51) Int. Cl.: C12P 1/04, C12N 1/20, C12N 15/63, C12N 9/04, C12P 7/46, C12P 7/52, C12N 15/52

(54) **RECOMBINANT MICROORGANISMS FOR PRODUCING ORGANIC ACIDS**
REKOMBINANTE MIKROORGANISMEN ZUR HERSTELLUNG VON ORGANISCHEN SÄUREN
MICROORGANISMES RECOMBINÉS POUR LA PRODUCTION D'ACIDES ORGANIQUES

(30) Priority: 02.10.2012 US 201261708998 P
(43) Date of publication of application: 12.08.2015
(73) Proprietor: The Michigan Biotechnology Institute, Lansing, Michigan 48910 (US)
(72) Inventor: GUETTLER, Michael, Holt, MI 48842 (US); HANCHAR, Robert, Charlotte, MI 48813 (US); KLEFF, Susanne, Okemos, MI 48864 (US); JADHAV, Sanchin, Lansing, MI 48910 (US)
(74) Representative: Patentanwälte Olbricht Buchhold Keulertz
(86) International application number: PCT/US2013/063100
(87) International publication number: WO 2014/055670

(56) References cited:
- WO-A2-2006/083410
- WO-A2-2012/103261
- LEE S J ET AL: "Genome-based metabolic engineering of Mannheimia succiniproducens for succinic acid production", APPLIED AND ENVIRONMENTAL MICROBIOLOGY, AMERICAN SOCIETY FOR MICROBIOLOGY, US, vol. 72, no. 3, 1 March 2006 (2006-03-01), pages 1939-1948, XP002581780, ISSN: 0099-2240, DOI: 10.1128/AEM.72.3.1939-1948.2006
- XINGJIANG LI ET AL: "Screening, breeding and metabolic modulating of a strain producing succinic acid with corn straw hydrolyte", WORLD JOURNAL OF MICROBIOLOGY AND BIOTECHNOLOGY, KLUWER ACADEMIC PUBLISHERS, DO, vol. 25, no. 4, 16 December 2008 (2008-12-16), pages 667-677, XP019690945, ISSN: 1573-0972
- YOUNG MI KIM ET AL: "Engineering the pentose phosphate pathway to improve hydrogen yield in recombinant Escherichia coli", BIOTECHNOLOGY AND BIOENGINEERING, vol. 108, no. 12, 19 December 2011 (2011-12-19), pages 2941-2946, XP055093808, ISSN: 0006-3592, DOI: 10.1002/bit.23259
- MARIA PAPAGIANNI: "Recent advances in engineering the central carbon metabolism of industrially important bacteria", MICROBIAL CELL FACTORIES, BIOMED CENTRAL, LONDON, NL, vol. 11, no. 1, 30 April 2012 (2012-04-30) , page 50, XP021126659, ISSN: 1475-2859, DOI: 10.1186/1475-2859-11-50
- VAN DER WERF M J ET AL: "Environmental and Physiological Factors affecting the succinate product ratio during carbohydrate ferentiation by Actinobacillus sp. 130Z", ARCHIVES OF MICROBIOLOGY, SPRINGER, DE, vol. 167, no. 6, 1 May 1997 (1997-05-01), pages 332-342, XP003003684, ISSN: 0302-8933, DOI: 10.1007/S002030050452

## Description

### Statement Regarding U.S. Government Support

Aspects of this invention were made with support of the United States government under the grant DE-FC36-02GO12001.

### Cross reference to Related Applications and Material Submitted Electronically

This application claims priority to U.S. Provisional Patent Application No. 61/708,998, filed October 2012.

This application containing, as a separate part of disclosure, a Sequence Listing in computer-readable form (filename: 40000A_SeqListing.txt, created 2 October 2013, 2006, 5 bytes).

### Technical Field of the Invention

The invention relates to bacteria demonstrating improved organic acid production and methods of use.

### Background of the Invention

Organic acids have potential for displacing petrochemically derived monomers in a range of industrial applications such as polymers, food, pharmaceuticals and cosmetics. The use of bio-based organic acids could decrease the reliance on fossil fuels and benefit the environment in terms of reduced carbon dioxide production.

WO 2006/083410 A2 discloses recombinant microorganisms for producing organic acids.

WO 2012/103261 A2 describes genetically modified yeast cells comprising an active succinate fermentation pathway, as well as methods of using these cells to produce succinate.

In the publication LEE, S J et al. Genome-based metabolic engineering of Mannheimia succiniproducens for succinic acid production. Appl Environ Microbiol. March 2006, Vol. 72 No. 3, pages 1939 to 48, gene knockout studies were carried out to understand the anaerobic fermentative metabolism of a succinic-acid-producing bacterium.

In the publication LI, X et al. Screening, breeding and metabolic modulating of a strain producing succinic acid with corn straw hydrolyte. World J. Microbiol. Biotechnol. December 2008, Vol. 25, No. 4, pages 667 to 677, it was shown that a succinic-acid-producing strain S.JST belonged to *Actinobacillus succinogenes* species.

The publication KIM Y M et al. Engineering the pentose phosphate pathway to improve hydrogen yield in recombinant Escherichia coli. Biotechnol. Bioeng. December 2011, Vol. 108, No. 12, pages 2941 to 2946, indicated that activation of the pentose phosphate pathway by glpX overexpression-enhanced gluconeogenic flux is implicated in the increase of NAD(P)H-dependent hydrogen production in E. coli BL21 (DE3).

In the publication PAPAGIANNI M. Recent advances in engineering the central carbon metabolism of industrially important bacteria. Microb. Cell Fact. April 2012, Vol. 11, No. 50, the optimization of existing or introduction of new cellular processes in microorganisms such as *Escherichia coli, Bacillus subtilis* and *Corynobacterium glutamicum* was discussed.

The publication VAN DER WERF M J et al. Environmental and physiological factors affected the succinate product ratio during carbohydrate fermentation by Actinobacillus sp. 130Z. Arch. Microbiol. May 1997, Vol. 167, Iss. 6, pages 332 to 342, introduced in examination proceedings, disclosed how ambient conditions could affect succinate formation.

### Summary of the Invention

Disclosed are recombinant bacteriafor producing organic acids. The disclosed recombinant bacteria(i) express heterologous glucose-6-phophate-1-dehydrogenase or overexpress glucose-6-phosphate dehydrogenase and (ii) express heterologous malate dehydrogenase or overexpress malate dehydrogenase, wherein the bacteria is a natural succinic acid producing bacteria, which results in improved organic acid production.

The following numbered paragraphs each succinctly define one or more exemplary variations of the invention:
1. A recombinant bacteria (i) expressing heterologous glucose-6-phosphate dehydrogenase or overexpressing glucose-6-phosphate dehydrogenase and (ii) expressing heterologous malate dehydrogenase or overexpressing malate dehydrogenase, wherein the bacteria is a natural succinic acid producing bacteria.
2. A recombinant bacteria comprising (i) a non-native or overexpressed polynucletide encoding a glucose-6-phosphate dehydrogenase and (ii) a non-native or overexpressed polynucleotide encoding a malate dehydrogenase.
3. The recombinant bacteriaof paragraph 1 or paragraph 2, comprising a 16S ribosomal RNA sequence with at least 90% identity to the 16S ribosomal RNA sequence of *Actinobacillus succinogenes.*
4. The recombinant bacteriaof paragraph 1 or paragraph 2, which is *Actinobacillus succinogenes, Bisgaard Taxon 6* or *Bisgaard Taxon 10.*
5. The recombinant bacteriaof any one of paragraphs 1-4, wherein the glucose-6-phosphate dehydrogenase or malate dehydrogenase are *A*. *succinogenes* enzymes.
6. The recombinant bacteriaof any one of paragraphs 1-4, wherein the glucose-6-phosphate dehydrogenase and malate dehydrogenase are *A*. *succinogenes* enzymes.
7. The recombinant bacteriaof any one of paragraphs 1-5, wherein the glucose-6-phosphate dehydrogenase or malate dehydrogenase are *E. coli* enzymes.
8. The recombinant bacteriaof any one of paragraphs 1-4, wherein the glucose-6-phosphate dehydrogenase and malate dehydrogenase are *E. coli* enzymes.
9. The recombinant bacteriaof any one of paragraphs 1-4, wherein the glucose-6-phosphate dehydrogenase comprises an amino acid sequence having at least about 40% sequence identity to the amino acid sequence set forth in SEQ ID NO: 2.
10. The recombinant bacteria of any one of paragraphs 1-4, wherein the malate dehydrogenase comprises an amino acid sequence having at least about 60% sequence identity to the amino acid sequence set forth in SEQ ID NO: 4.
11. The recombinant bacteria of any one of paragraphs 1-10, wherein the bacteria demonstrates improved organic acid (e.g., succinic acid) production compared to a bacteria expressing glucose-6-phosphate dehydrogenase or malate dehydrogenase enzymes alone.
12. The recombinant bacteria of any one of paragraphs 1-11, wherein the bacteria is capable of producing succinic acid at concentrations of about 50g/L to 150g/L.
13. A process for organic acid production, which process comprises culturing the recombinant bacteria of any one of paragraphs 1-12 in a fermentation medium.
14. A method of producing organic acid comprising the step of culturing a recombinant bacteria expressing glucose-6-phosphate dehydrogenase and malate dehydrogenase enzymes with a carbon source under conditions favoring organic acid production, wherein the enzymes are not native to the bacteria or are overexpressed compared to an unmodified parent bacteria.
15. A method of producing organic acid comprising culturing the recombinant bacteria of any one of paragraphs 1-12 with a carbon source under conditions favoring organic acid production.
16. The method of any one of paragraphs 13-15, wherein the organic acid is succinic acid or propionic acid.
17. The method of paragraph 16, wherein the organic acid is succinic acid.
18. The method of any one of paragraphs 14-17, wherein the carbon source is glucose or polyol.

### Brief Description of the Drawings

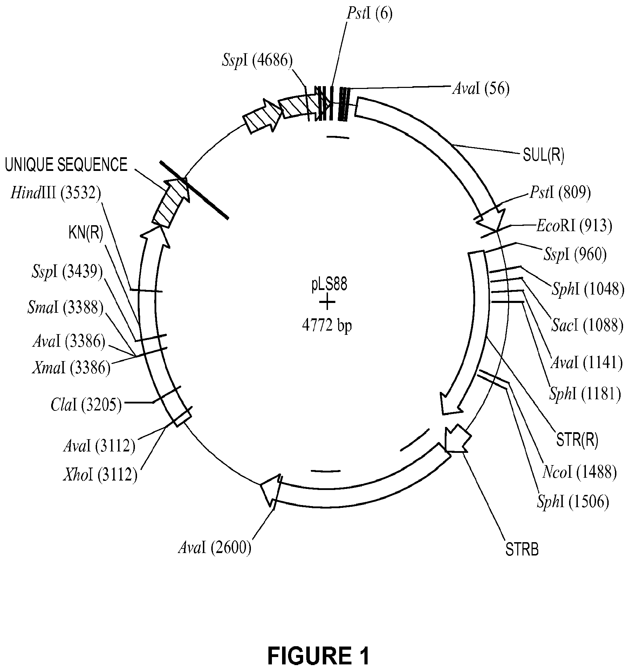

FIG. 1 depicts a schematic map of pL88 vector.
FIG. 2 depicts a schematic map of pISTONS 1.
FIG. 3 depicts a schematic map of p830.60.

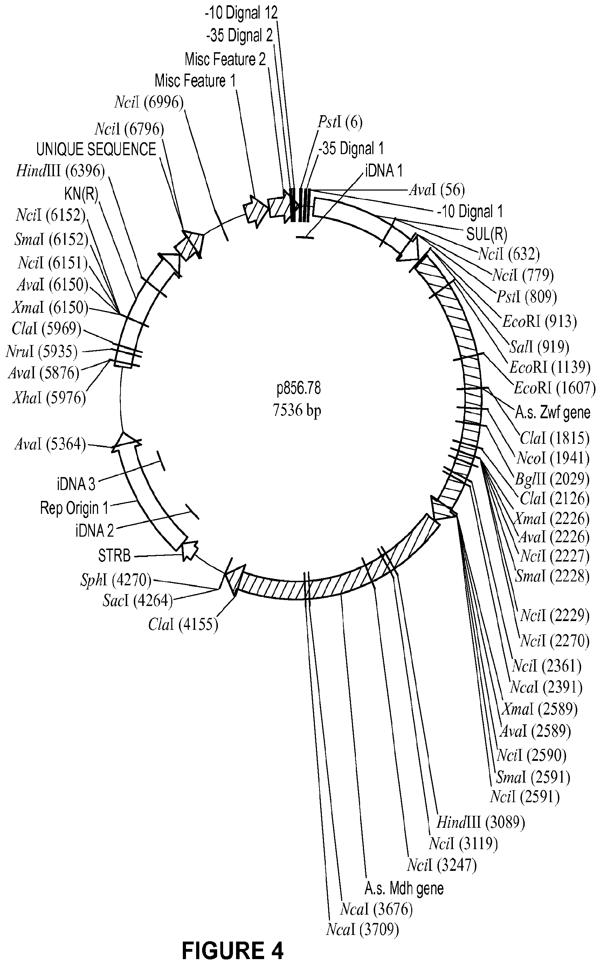

FIG. 4 depicts a schematic map of p856.78.
FIG. 5 is a sequence comparison between the amino acid sequences of the Zwf enzymes from *E. coli* ("query") and *A*. *succinogenes* ("sbjct").
FIG. 6 is a sequence comparison between the amino acid sequences of the Mdh enzymes from *E. coli* ("query") and *A*. *succinogenes* ("sbjct").
FIG. 7 is a sequence comparison between the amino acid sequences of the Mdh enzyme from *Aspergillus flavus* ("query") and *A*. *succinogenes* ("sbjct").
FIG. 8 is an A. succinogenes zwf nucleic acid sequence in p830.60.
FIG. 9 is an A. succinogenes mdh nucleic acid sequence in pISTONS1.

### Detailed Description of the Invention

In the following detailed description, embodiments are described in sufficient detail to enable those skilled in the art to practice them.

The term "recombinant bacteria" refers to a bacteria altered, modified or engineered (e.g., genetically engineered) such that it exhibits an altered, modified or different genotype or phenotype (e.g., when the genetic modification affects coding nucleic acid sequences of the bacteria) as compared to the naturally-occurring or starting bacteria from which it was derived.

Genetic manipulation can include, but is not limited to, altering or modifying regulatory sequences or sites associated with expression of a particular gene (e.g., by using strong promoters, inducible promoters or multiple promoters); modifying the chromosomal location of a particular gene; altering nucleic acid sequences adjacent to a particular gene, such as a sequence in the promoter region including regulatory sequences important for the promoter activity, a ribosome binding site, or transcription terminator; increasing the copy number of a particular gene; modifying proteins (e.g., regulatory proteins, suppressors, enhancers, transcriptional activators, and the like) involved in transcription or translation of a particular gene product; or any other conventional means of increasing expression of a particular gene.

As used herein, an "organic acid" includes an acid comprising at least one carboxylic group. Optionally, the organic acid is a C₁-C₁₀ organic acid, also optionally the organic acid comprises two carboxylate groups. For example, "organic acid" includes succinic acid or propionic acid. Other examples of organic acids include, but are not limited to, lactic acid, malic acid, citric acid, oxalic acid, and tartaric acid. As used herein, organic acids may also include the organic acid anion or a salt thereof. For example, "succinic acid" may be referred to as "succinate;" "propionic acid" may be referred to as "propionate."

"Heterologous" refers to any polynucleotide or polypeptide that does not originate or is not native to the particular cell or organism where expression is desired.

"Overexpression" refers to expression of a polynucleotide to produce a product (e.g., a polypeptide or RNA) at a higher level than the polynucleotide is normally expressed in the host cell. An overexpressed polynucleotide is generally a polynucleotide native to the host cell, the product of which is generated in a greater amount than that normally found in the host cell. Overexpression is achieved by, for instance and without limitation, operably linking the polynucleotide to a different promoter than the polynucleotide's native promoter or introducing additional copies of the polynucleotide into the host cell.

A polypeptide or polynucleotide "derived from" an organism comprises the same amino acid sequence or nucleic acid sequence as the reference polypeptide or polynucleotide from the organism, or optionally contains one or more modifications to the native amino acid sequence or nucleotide sequence, as described further below, and optionally exhibits similar, if not better, activity compared to the native enzyme (e.g., at least 70%, at least 80%, at least 90%, at least 95%, at least 100%, or at least 110% the level of activity of the native enzyme). It will be appreciated that a polypeptide or polynucleotide "derived from" an organism is not limited to polypeptides or polynucleotides physically removed from a particular host organism.

Also herein, the recitations of numerical ranges by endpoints include all numbers subsumed within that range (e.g., 1 to 5 includes 1, 1.5, 2, 2.75, 3, 3.80, 4, 5, and the like). Furthermore, disclosure of a range includes disclosure of all subranges included within the broader range (e.g., 1 to 5 discloses 1 to 4, 1.5 to 4.5, 4 to 5, and the like).

The invention provides improved materials and methods for producing organic acid via fermentation. Fermentation performance is typically captured by three parameters: titer, productivity, and yield. The titer specifies the concentration of the product (e.g., organic acid, such as succinic acid) in the fermentation broth at the end of the fermentation. The productivity or rate of production represents the time to reach the titer of product. Yield is the quotient of mass of product per mass of feedstock (e.g., sugar, such as glucose). All three parameters affect the economics of a fermentative production process. A high titer facilitates product recovery; less water must be evaporated or removed, requiring less energy, which lowers the operating costs of the process. The productivity is linked to titer, adding a time dimension to it. High productivity implies a fast fermentation processes, and allows a reduction in fermentation vessel size, since the process can be run more often. Productivity affects mainly the capital cost of fermentative production processes. The yield affects the operating costs of the process - the higher the yield, the less raw material or feedstock is needed to obtain the product.

Titer, productivity, and yield are intertwined and affect one another. A fermentation that achieves a high titer in a short time will result in a high productivity and *vice versa.* The accumulation of a fermentation product or higher titer can slow production. Yield is inversely correlated to productivity, in that feedstock is used in the fermentation to support growth of the organism, multiplying of cells and building biomass, but also to metabolize feedstock into the desired product. Generally, more cells will produce more product and will produce it faster, which will result in higher productivity, but lower yield. Hence, fermentation optimization must balance all three parameters to achieve the most economical process. The ultimate goal is to achieve a process that has high values for all three parameters.

Remarkably, simultaneous over-production of enzymes in the pentose phosphate pathway (or Entner-Doudoroff pathway) and tri-carboxylic acid cycle in a recombinant microbe (e.g., *Actinobacillus succinogenes*) as described herein was discovered to promote a higher yield of succinic acid, which is a key factor in determining the economic viability of the fermentation process. In this regard, the invention is predicated, at least in part, on the surprising discovery that production of a glucose-6-phosphate dehydrogenase and a malate dehydrogenase in a bacteria results in improved organic acid production compared to the level of production achieved by a bacteria expressing a single enzyme alone. The pentose phosphate cycle (or Entner-Doudoroff pathway) utilizes several enzymes including glucose-6-phosphate dehydrogenase (e.g., glucose-6-phosphate-1-dehydrogenase, which is also referred to as Zwischenferment enzyme or Zwf); 6-phosphogluconolactonase; 6-phosphogluconate dehydrogenase, (also called Gnd); ribose-5-phosphate isomerase A and B; ribulose phosphate 3-epimerase; transketolase I and II; transaldolase A and B; 6-phosphogluconate dehydratase (Edd); and 2-keto-3-deoxyphosphogluconate aldolase (Eda). The tri-carboxylic acid cycle used in the anaerobic production of succinic acid uses, e.g., the enzymes phosphoenolpyruvate-carboxykinase, malate dehydrogenase, fumarase, and fumarate-reductase.

In one aspect, the invention provides a recombinant bacteria that (i) expresses a heterologous glucose-6-phosphate dehydrogenase (e.g., Zwf) or overexpresses glucose-6-phosphate dehydrogenase and (ii) expresses a heterologous malate dehydrogenase (e.g., Mdh) or overexpresses malate dehydrogenase, wherein the bacteria is a natural succinic acid producing bacteria, such that, in various embodiments, the recombinant bacteria exhibits enhanced organic acid (e.g., succinic acid or propionic acid) production compared to an unmodified parent bacteria. The activities of glucose-6-phosphate dehydrogenase (EC 1.1.1.49) and malate dehydrogenase (EC 1.1.1.37) are well understood in the art. Glucose-6-phosphate dehydrogenase catalyzes, for example, the reaction D-glucose-6-phosphate + NADP+ => 6-phospho-D-glucono-1,5-lactone + NADPH. Malate dehydrogenase converts oxalo-acetate to malate, or oxaloacetic acid to malic acid, reducing the substrate through the use of co-factors such as, but not limited to, NADH or NADPH.

Glucose-6-phosphate dehydrogenase and malate dehydrogenase are found in a wide variety of organisms. One or both of the enzymes may be native to the recombinant bacteria, but multiple copies of a polynucleotide encoding the enzyme are introduced into the host bacteria cell to increase production of the enzyme. In some embodiments, the recombinant bacteria expresses a native glucose 6-phosphate dehydrogenase and a native malate dehydrogenase at elevated levels (e.g., "overexpress" the enzyme) relative to levels present in non-recombinant bacteria or the starting bacteria. Alternatively, one or both of the enzymes is not native to the recombinant bacteria. In various embodiments, the polynucleotides are derived from an organism that naturally produces succinic acid or malic acid. Depending on the embodiment of the invention, the polynucleotide is isolated from a natural source such as bacteria, algae, fungi, plants, or animals; produced via a semi-synthetic route (e.g., the nucleic acid sequence of a polynucleotide is codon-optimized for expression in a particular host cell, such as *E. coli*); or synthesized *de novo.*

Polynucleotides encoding malate dehydrogenase (e.g., *mdh* genes) may be derived, for example, from *A*. *succinogenes* (e.g., NC_009655) or *E. coli* (EG10576 (EcoCyc)), or from other suitable sources shown to have the enzymatic activity. Similarly, the polynucleotide encoding the glucose 6-phosphate dehydrogenase is, in various embodiments, derived from *E. coli.* (e.g., EG11221 (EcoCyc); Accession Number: ECK1853) or A. *succinogenes* (e.g., GenBank Accession No. ABR73607.1 GI:150839636). Optionally, polynucleotides are codon optimized to facilitate expression in a particular bacteria. In some embodiments, the recombinant bacteria comprises a polynucleotide comprising the nucleic acid sequence of SEQ ID NO: 1, which encodes Zwf enzyme, and/or a polynucleotide comprising the nucleic acid sequence encoding SEQ ID NO: 3, which encodes an Mdh enzyme. Also contemplated herein are polynucleotides comprising a nucleic acid sequence that is at least 50%, at least 55%, at least 60%, at least 65%, at least 70%, at least 75%, at least 80%, at least 85%, at least 90%, or at least 95% (or any range of the foregoing percentages) to SEQ ID NO: 1 or SEQ ID NO: 3.

In some embodiments, the variant polynucleotide encodes a polypeptide having one or more biochemical activities of Zwf or Mdh enzyme. A variant polynucleotide may include, for example, a fragment of the *zwf or mdh* gene. In some embodiments, the recombinant bacteria may express a *zwf or mdh* gene expressed by A. *succinogenes, E. coli, Basfia* (e.g., *Basfia succinicproducens), Mannheimia* (e.g., *Mannheimia succiniciproducens), Saccharomyces, Aspergillus* or a variant thereof.

The inventors have discovered that (i) expression of heterologous Zwf or the overexpression of Zwf and (ii) expression of heterologous Mdh or overexpression of Mdh enzymes in a single fermentation process, wherein the bacteria is a natural succinic acid producing bacteria, results in an improved organic acid production such as succinic acid or propionic acid. According to the invention, a single recombinant bacteria is used that coexpresses Zwf and Mdh. The invention contemplates a recombinant bacteria comprising (e.g., a bacteria that has been transformed with) a polynucleotide encoding a polypeptide having one or more biochemical activities of the Mdh enzyme in combination with a polynucleotide encoding a polypeptide having one or more biochemical activities of the Zwf enzyme, such as glucose-6-phosphate dehydrogenase activity and/or NADP reductase activity. For example, a suitable Zwf or Mdh enzyme is the *E. coli* Zwf or Mdh enzyme or a variant thereof. Put another way, in various embodiments, the glucose-6-phosphate dehydrogenase and/or malate dehydrogenase are/is *E. coli* or *A*. *succinogenes* enzymes.

A representative glucose 6-phosphate dehydrogenase amino acid sequence is provided in SEQ ID NO: 2, which is an *A*. *succinogenes* Zwf amino acid sequence. The recombinant bacteria may express a variant polypeptide having at least about 40% sequence identity to the amino acid sequence of a Zwf enzyme (e.g., SEQ ID NO: 2), and more desirably at least about 50%, at least about 60%, at least about 70%, at least about 80%, or at least about 90% sequence identity to the amino acid sequence of a Zwf enzyme (e.g., SEQ ID NO: 2). In various embodiments, the variant polypeptide comprises one or more conservative mutations with respect to a reference sequence, i.e., a substitution of an amino acid with a functionally similar amino acid. Put another way, a conservative substitution involves replacement of an amino acid residue with an amino acid residue having a similar side chain. Families of amino acid residues having similar side chains have been defined within the art, and include amino acids with basic side chains (e.g., lysine, arginine, and histidine), acidic side chains (e.g., aspartic acid and glutamic acid), uncharged polar side chains (e.g., glycine, asparagine, glutamine, serine, threonine, tyrosine, and cysteine), nonpolar side chains (e.g., alanine, valine, leucine, isoleucine, proline, phenylalanine, methionine, and tryptophan), beta-branched side chains (e.g., threonine, valine, and isoleucine) and aromatic side chains (e.g., tyrosine, phenylalanine, tryptophan, and histidine).

A representative malate dehydrogenase amino acid sequence is provided in SEQ ID NO: 4, which is an *A*. *succinogenes* Mdh amino acid sequence. The recombinant bacteria may express a variant polypeptide having at least about 60% sequence identity to the amino acid sequence of a Mdh enzyme (e.g., SEQ ID NO: 4), and more desirably at least about 70% or 80% or 90%, sequence identity to the amino acid sequence of a Mdh enzyme (e.g., SEQ ID NO: 4).

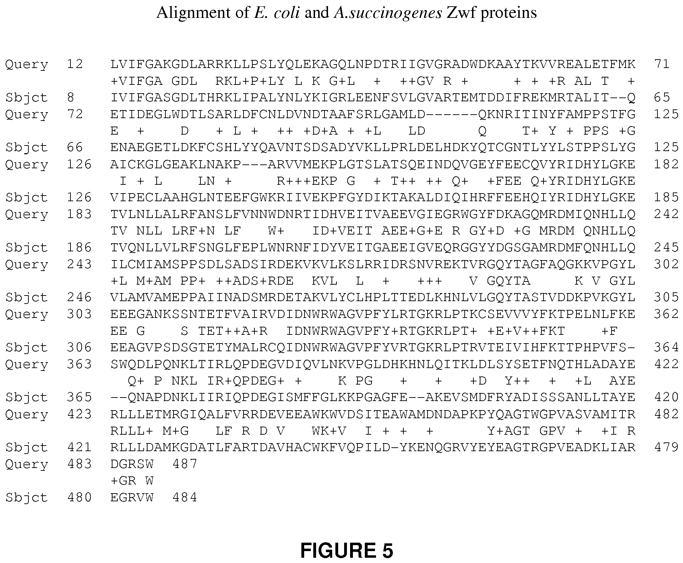

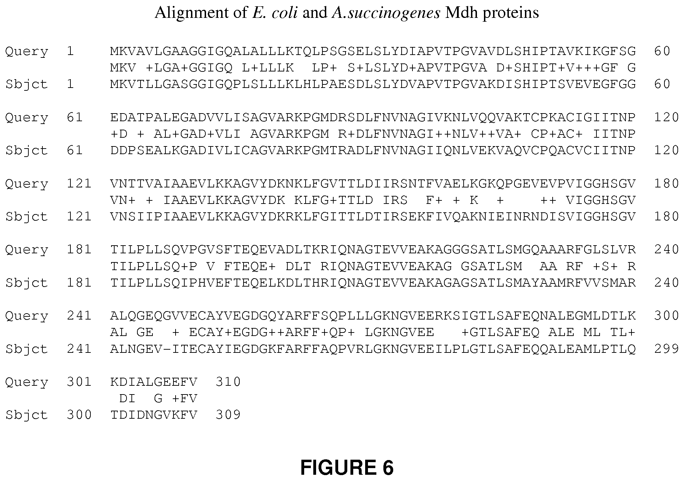

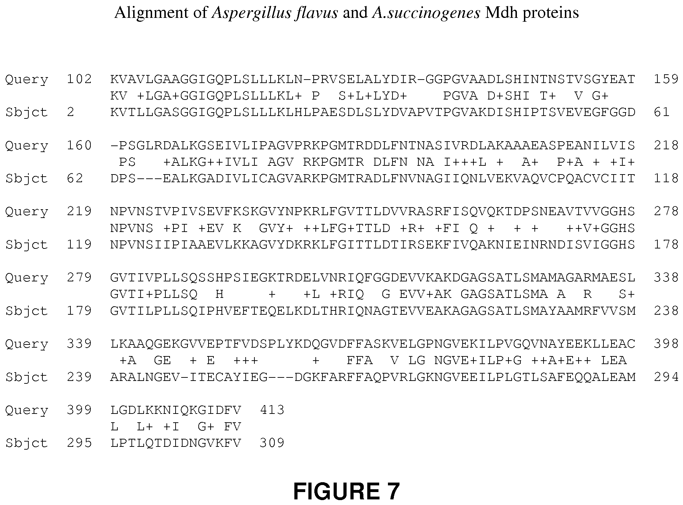

Substantial variation within the amino acid sequence is permitted in the context of the enzymes of the recombinant bacteria. The amino acid sequences of the Zwf enzymes from *E. coli* and *A*. *succinogenes,* for instance, share 44% identity, and both are suitable enzymes in the context of the invention. See Fig. 5. Similarly, the amino acid sequences of Mdh enzymes from *E. coli* and *A. succinogenes* share 69% identity, and both are suitable enzymes in the context of the invention. See Fig. 6. The Mdh enzyme is generally well conserved among different organisms. The amino acid sequence of the Mdh enzyme from *Aspergillus flavus,* a filamentous fungus, demonstrates about 50% identity to the Mdh enzyme sequence from *A. succinogenes,* and retains the ability to convert oxalo-acetate to malate. See Fig. 7.

Desirably, the variant polypeptide has one or more biochemical activities of the Zwf or Mdh enzyme, e.g., glucose-6-phosphate dehydrogenase and malate dehydrogenase activity. A variant polypeptide may include a fragment of the Zwf or Mdh enzyme. Methods of evaluating glucose-6-phosphate dehydrogenase activity and malate dehydrogenase activity are known in the art and available commercially from, e.g., Abcam, Cambridge, MA, and Sigma Aldrich, St. Louis, MO. An exemplary assay for determining glucose-6-phosphate dehydrogenase activity is described in Rowley and Wolf, J. Bacteriology, 173, 968-977 (1991) and Wolf et al., J. Bacteriology, 139, 1093-1096 (1979). Illustrative conditions for the reaction are as follows: a reaction mixture containing 50 mM Tris-HCl (pH 7.8), 10 mM MgCl₂, 1 mM NADP, 1 mM glucose-6-phosphate, and cell extract is prepared, and absorbance increase at 340 nm is measured. In various embodiments, the glucose-6-phosphate dehydrogenase encoded by the polynucleotide exhibits at least 70%, at least 75%, at least 80%, at least 85%, at least 90%, at least 95%, or 100% of the glucose-6-phosphate dehydrogenase activity of the polypeptide of SEQ ID NO: 2. The glucose-6-phosphate dehydrogenase encoded by the polynucleotide may exhibit greater than 100% of the glucose-6-phosphate dehydrogenase activity of the polypeptide of SEQ ID NO: 2 (e.g., 110% or more, 120% or, or 130% or more of the activity). Alternatively or in addition, the malate dehydrogenase produced by the recombinant bacteria exhibits at least 70%, at least 75%, at least 80%, at least 85%, at least 90%, at least 95%, or 100% of the malate dehydrogenase activity of the polypeptide of SEQ ID NO: 4. The malate dehydrogenase encoded by the polynucleotide may exhibit greater than 100% of the malate dehydrogenase activity of the polypeptide of SEQ ID NO: 4 (e.g., 110% or more, 120% or, or 130% or more of the activity). An exemplary assay for determining malate dehydrogenase activity is described in Samuelov, J. Bacteriology, 57, 3013 (1991). Illustrative conditions for the reaction are as follows: a reaction mixture containing 100 mM Tris-HCl, pH 8.1, 5 mM oxaloacetate, 0.3 mM NADH, 2 mM DTT, and cell extract is prepared; and absorbance decrease at 340 nm, representing oxidation of NADH, is measured.

The recombinant bacteria may be derived from any suitable bacterial cells. The bacteria is capable of producing an organic acid at a level suitable for commercial production. Suitable bacteria for preparing recombinant bacteria as described herein include succinic acid producing bacteria. Exemplary bacteria include, but are not limited to, bacteria of the *Pasteurellaceae* family. Examples of *Pasteurellaceae* family members include members of the *Actinobacillus* genus, including *A. succinogenes, Bisgaard Taxon 6* (deposited with the Culture Collection, University of Goteborg, Sweden (CCUG), under accession number 15568), *Bisgaard Taxon 10* (deposited under CCUG accession number 15572), *Mannheimia succiniciproducens, Basfia succinicproducens, E. coli, Anaerobiospirillum succiniciproducens, Ruminobacter amylophilus, Succinivibrio dextrinosolvens, Prevotella ruminicola, Ralstonia eutropha,* and coryneform bacteria (e.g., *Corynebacterium glutamicum, Corynebacterium ammoniagenes, Brevibacterium flavum, Brevibacterium lactofermentuin, Brevibacterium divaricatum);* members of the *Lactobacillus genus;;* and any subset thereof. In some embodiments, the recombinant strain may be (is) derived from a bacteria whose 16S rRNA has at least 90% sequence identity (e.g., at least 95% sequence identity) to 16S rRNA of *Actinobacillus succinogenes.* For example, the recombinant strain may be derived from a strain of *Actinobacillus succinogenes, Bisgaard Taxon 6, or Bisgaard Taxon 10.*

The recombinant bacteria expresses a *zwf gene* and a *mdh* gene. The *zwf or mdh* genes may be native to the recombinant bacteria and overexpressed. In other embodiments, the *zwf or mdh* genes may be heterologous (i.e., not native to the bacteria from which the recombinant bacteria is derived). In some embodiments, the polynucleotide encoding the glucose-6-phosphate dehydrogenase and/or the polynucleotide encoding the malate dehydrogenase (e.g., the *zwf or mdh* gene) are optimized for expression in the recombinant bacteria. For example, *zwf or mdh* genes may be operationally linked to a promoter that facilitates gene overexpression relative to a non-recombinant bacteria. The promoter may be endogenous to the bacteria (e.g., native to the bacteria from which the recombinant bacteria is derived) or heterologous to the bacteria. In some embodiments, the *zwf* and *mdh* genes are from *A. succinogenes* and are regulated by their respective *zwf* and *mdh* promoters also from *A. succinogenes.*

The polynucleotide encoding the glucose-6-phosphate dehydrogenase and/or the polynucleotide encoding the malate dehydrogenase (e.g., *zwf* or *mdh* genes) are optionally modified to facilitate translation of the corresponding mRNA. For example, a *zwf or mdh* gene may be modified to include codons that are not present in the endogenous or native gene. These non-endogenous codons may be selected to reflect the codon usage frequency in the recombinant bacteria. Codon usage tables have been developed for many bacteria and are known in the art. For example, the polynucleotide encoding the glucose-6-phosphate dehydrogenase and/or the polynucleotide encoding the malate dehydrogenase (e.g., *zwf* or *mdh* genes) may be modified to reflect the codon usage frequency for *A. succinogenes* as provided in U.S. Patent No. 8,119,377.

The recombinant bacteria may be derived from a strain that produces high levels of one or more organic acids such as succinic acid or propionic acid, or the recombinant bacteria may be selected or engineered to produce high or enhanced levels of one or more organic acids such as succinic acid or propionic acid relative to a non-recombinant bacteria.

Optionally, the recombinant bacteria is selected or engineered (or both) to be resistant to relatively high levels of undesirable by-products or to produce relatively low levels of undesirable by-products. For example, after introduction of the polynucleotides encoding the glucose-6-phosphate dehydrogenase and malate dehydrogenase into the cell (e.g., transformation), a population of recombinant bacteria is optionally grown in the presence of sodium monofluoroacetate to select strains that are resistant to relatively high acetate levels or strains that produce relatively low acetate levels. Undesirable by-products include, but are not limited to, formate (or formic acid), acetate (or acetic acid), and/or pyruvate (or pyruvic acid), lactate, 1.3-propanediol, and ethanol. Methods for selecting strains that produce low acetate levels are known in the art. See, e.g., U.S. Patent No. 5,521,075 and U.S. Patent No. 5,573,931, particularly with respect to their discussion of microbial strain selection. For example, strains of bacteria that produce relatively low acetate levels may be selected by growing the bacteria in the presence of a toxic acetate derivative, such as sodium monofluoroacetate at a concentration of about 1.0 to about 8.0 g/L. Selected strains may produce relatively low acetate levels (e.g., less than about 10 g/L, less than about 7 g/L, less than about 5 g/L, or less than about 2.0 g/L), formate (e.g., less than about 2.0 g/L), and/or pyruvate (e.g., less than about 3.0 g/L) in a glucose fermentation. One suitable monofluoroacetate resistant strain for producing a recombinant bacteria or derivative is a strain of A. *succinogenes* deposited under ATCC accession number 55618. See also U.S. Patent No. 5,573,931, which describes suitable methods for preparing microbial strains that are resistant to monofluoroacetate. Other suitable monofluoroacetate resistant strain examples include FZ45 and FZ53, also described in U.S. Patent No. 5,573,931.

A polynucleotide encoding a glucose-6-phosphate dehydrogenase and a polynucleotide encoding a malate dehydrogenase (or a polypeptide with one or more biochemical activities of the Zwf or Mdh enzyme) may be obtained by employing methods known in the art (e.g., PCR amplification with suitable primers and cloning into a suitable DNA vector). The polynucleotide sequences of suitable *zwf* genes encoding glucose-6-phosphate dehydrogenase activity have been disclosed. (See, e.g., GenBank). For example, the polynucleotide sequence of the A. *succinogenes zwf gene* has been published in U.S. Patent No. 8,119,377, which is hereby incorporated by reference. See also Joint Genome Institute, Department of Energy website, NCBI accession number NC_009655. The *E. coli zwf gene* is deposited with GenBank (e.g., under GenBank Accession Number NC 000913 and GenBank Accession Number M55005). The *zwf gene* or variants thereof may be obtained by PCR amplification of a bacteria's genomic DNA with appropriate primers.

The *mdh* sequences may be obtained, for example, from *Actinobacillus succinogenes,* GenBank NC_009655, or *E. coli.* EG10576 (EcoCyc).

The DNA vector may be any suitable vector for expressing polynucleotide(s) in a recombinant bacteria. Suitable vectors include plasmids, bacterial artificial chromosomes), and/or modified bacteriophages (e.g., phagemids). The vector may be designed to exist as an epigenetic element and/or the vector may be designed to recombine with the bacteria's genome.

The polynucleotide typically includes a promoter operationally linked to a nucleic acid sequence that encodes a glucose-6-phosphate dehydrogenase or malate dehydrogenase (i.e., a polypeptide having Zwf or Mdh enzyme activity). The promoter may be endogenous or native to the bacteria from which the recombinant bacteria is derived or heterologous to the bacteria (e.g., derived from a source other than the recombinant bacteria). Furthermore, the promoter may be the native promoter for a selected glucose-6-phosphate dehydrogenase or malate dehydrogenase coding sequence (i.e., *zwf or mdh* gene) or may be a promoter other than the native promoter for a selected glucose-6-phosphate dehydrogenase or malate dehydrogenase (e.g., a non-*zwf* or *mdh* gene promoter). In some embodiments, the recombinant bacteria is a *A. succinogenes* strain, and the *A. succinogenes zwf or mdh* promoter is used. In other embodiments, the promoter may be an *A. succinogenes* phosphoenolpyruvate *(PckA)* carboxykinase promoter, deposited under GenBank accession number AY308832, including nucleotides 1-258, or a variant thereof, as disclosed in U.S. Patent No. 8,119,377. Any suitable promoter (e.g., inducible, constitutive, strong and the like) that can direct expression of the desired sequence in the desired bacteria may be used. The promoter may be operationally linked to the polynucleotide encoding glucose-6-phosphate dehydrogenase or polynucleotide encoding malate dehydrogenase (e.g., *zwf or mdh* gene) using cloning methods that are known in the art. For example, the promoter and the coding sequence (e.g. *zwf* and *mdh* gene) may be amplified by PCR using primers that include compatible restriction enzyme recognition sites. The amplified promoter and coding sequence then may be digested with the enzyme and cloned into an appropriate vector that includes a suitable multiple cloning site.

In addition, the polynucleotide(s) or vector may include or encode a selectable marker. The selectable marker may impart resistance to one or more antibiotic agents. For example, selectable markers may include genes for ampicillin resistance, streptomycin resistance, kanamycin resistance, tetracycline resistance, chloramphenicol resistance, sulfonamide resistance, or combinations of these markers. Typically, the selectable marker is operationally linked to a promoter that facilitates expression of the marker. Plasmids and other cloning vectors that include selectable markers are known in the art.

Any suitable host cell may be used to store or propagate a vector comprising the polynucleotide(s). The host cell optionally expresses a coding sequence on the DNA molecule. Suitable host cells also may include cells that are capable of producing (i.e., cells that produce) an organic acid in a fermentation process, such as succinic acid or propionic acid at a concentration suitable for commercial production (e.g., at least about 50 g/L, and more suitably at least about 100 g/L).

In the context of the invention, methods for producing an organic acid typically include fermenting a nutrient medium with a recombinant bacteria. The recombinant bacteria in the fermentation medium expresses a *zwf* and *mdh* gene. The method may include fermenting a nutrient medium with a recombinant A. *succinogenes* that expresses a *zwf* and *mdh* gene (e.g., a native A. succinogenes *zwf or mdh* gene. It can be envisioned that other combinations of genes and organisms may be used to ferment medium to produce organic acid. For example a heterologous *zwf* gene and endogenous *mdh* gene may be both expressed from a heterologous promoter in a suitable bacteria or any other suitable combinations.

Thus, the invention provides a method of producing organic acid comprising culturing the recombinant bacteria described herein with a carbon source under conditions favoring organic acid production. The recombinant bacteria, for example, comprises a non-native or overexpressed polynucleotide encoding a glucose-6-phosphate dehydrogenase enzyme and a non-native or overexpressed polynucleotide encoding a malate dehydrogenase enzyme, and produces the enzymes to enable production of a desired organic acid. Organic acids produced in the fermentation may include, for example, succinic acid or propionic acid, or any other organic acid discussed herein.

One suitable recombinant bacteria for the methods described herein is a recombinant strain of *A. succinogenes* that expresses the *E. coli zwf* gene, deposited under ATCC accession number PTA-6255. This organism, in combination with another recombinant organism expressing the *mdh* gene, may be both grown on fermenting nutrient medium to produce organic acid. Alternatively, the strain deposited under ATCC accession number PTA-6255 is modified to overexpress *mdh* or produce heterologous Mdh. The methods also may include fermenting a nutrient medium with a recombinant strain of *Bisgaard Taxon* 6 or *Bisgaard Taxon* 10 that express a *zwf or mdh* or both genes (e.g., a heterologous *zwf or mdh* gene such as from *E. coli)* to produce succinic acid.

The nutrient medium typically includes a fermentable carbon source. The fermentable carbon source may be provided by a fermentable biomass. A fermentable biomass may be derived from a variety of crops and/or feedstocks including: sugar crops (e.g., sugar, beets, sweet sorghum, sugarcane, fodder beet); starch crops (e.g., grains such as corn, wheat, sorghum, barley, and tubers such as potatoes and sweet potatoes); cellulosic crops (e.g., corn stover, corn fiber, wheat straw, and forages such as Sudan grass forage, and sorghum). The biomass may be treated to facilitate release of fermentable carbon source (e.g., sugars). For example, the biomass may be treated with enzymes such as cellulase and/or xylanase, to release simple sugars, and/or may be treated with heat, steam, or acid to facilitate degradation. The fermentable carbon source may include simple sugars and sugar alcohols such as glucose, maltose, mannose, mannitol, sorbitol, galactose, xylose, xylitol, arabinose, arabitol, and mixtures thereof. In some embodiments, non-purified, minimally processed or crude carbon sources may be used for organic acid production. In one embodiment, the carbon source may include crude polyols described in U.S. Patent Application Serial No. 60/709,036, entitled "Integrated Systems & Methods for Organic Acid Productions" filed on the same date as the U.S. Provisional Patent Application No. 61/708998, to which the instant application claims priority, which application is incorporated by reference herein in its entirety. Crude polyols include, for example, polyols produced via, e.g., fermentation or hydrogenation, which are otherwise subjected to minimal processing. For example, crude polyols are not subjected to a filtration step to remove solids. As such, crude polyols are less pure than refined polyols.

The methods of the invention result in a relatively high yield of succinic acid relative to an input carbon source, such as sugar (e.g., glucose or sorbitol). For example, the methods optionally have a succinic acid yield (g) of at least about 70%, at least about 75%, at least about 80%, at least about 85%, at least about 90%, or at least about 95% (or any range of the foregoing percentages) relative to carbon source (e.g., glucose) input (g), or a propionic acid yield (g) of at least 56% (e.g., at least about 60%, at least about 65%, at least about 70%, at least about 75%, at least about 80%, at least about 85%, at least about 90%, or at least about 95%, or any range of the foregoing percentages) relative to carbon source (e.g., glucose) input (g). In some embodiments, the yield may be calculated as % succinic acid or propionic acid yield (mol)/ carbon source (e.g., glucose) input (mol). As such, the methods may have a succinic acid or propionic yield (mol) of at least about 154% relative to carbon source (e.g., glucose) input (mol). Desirably, the methods may have a succinic acid or propionic acid yield (mol) of at least about 130% or at least about 170% relative to carbon source (e.g., glucose) input (mol).

The methods may result in a relatively high succinic acid concentration (e.g., relative to a method that uses a non-recombinant bacteria in a fermentation). For example, a fermentation may reach a concentration of at least about 50 g/L succinic acid (e.g., at least about 60 g/L, at least about 70 g/L, or at least about 80 g/L). Desirably, a fermentation may reach a concentration of at least about 90 g/L succinic acid (e.g., at least about 100 g/L succinic acid, at least about 110 g/L succinic acid, or at least about 120 g/L succinic acid) or more desirably, a concentration of at least about 130 g/L succinic acid and even more desirably a concentration of at least about 140 g/L. In some embodiments, the fermentation typically does not produce substantial levels of undesirable by-products such as acetate, formate, pyruvate, and mixtures thereof (e.g., no more than about 11.0 g/L acetate, no more than about 10.0 g/L acetate, no more than about 9.0 g/L acetate, no more than about 8.0 g/L acetate, no more than about 7.0 g/L acetate, no more than about 6.0 g/L acetate, no more than about 5.0 g/L acetate, no more than about 4.0 g/L acetate, no more than about 3.0 g/L, or no more than about 2.0 g/L acetate (e.g., 1.5 g/L or less or 1.0 g/L or less); no more than about 2.0 g/L formate (e.g., 1.5 g/L or less or 1.0 g/L or less); and/or no more than about 3.0 g/L pyruvate (e.g., 2.5 g/L or less or 2.0 g/L or less or 1.5 g/L or less or 1.0 g/L or less)).

The method described herein optionally results in relatively high production rates. In various embodiments, the method achieves a productivity of at least about 1.0 g/L-h, at least about 1.5g/L-h, at least about 2.0 g/L-h, at least about 2.5 g/L-h, at least about 3.0 g/L-h, at least about 3.5 g/L-h, or at least about 4.0 g/L-h.

In one embodiment, the recombinant bacteria is *Actinobacillus succinogenes* that expresses a heterologous *zwf* and *mdh* gene. The heterologous *zwf* and *mdh* genes may be optimized for expression in *Actinobacillus succinogenes.* The heterologous *zwf* and *mdh* genes may be encoded by *E. coli.* For example, a recombinant organism is *Actinobacillus succinogenes* deposited under ATCC Accession Number PTA-6255 (American Type Culture Collection, Manassas, VA, USA), which is optionally modified to overproduce Mdh. An alternative recombinant bacteria is an *A. succinogenes* strain deposited under ATCC Accession Number PTA-120462, which is a strain that produces increased levels of Zwf and Mdh compared to a parent (unmodified) *A. succinogenes* strain. The recombinant strain may be capable of producing succinic acid or propionic acid at concentrations of about 50 g/L to about 150 g/L (e.g., in a fermentation system that utilizes a suitable carbon source). The recombinant strain may be resistant to levels of sodium monofluoroacetate of at least about 1 g/L.

In another embodiment, the recombinant strain is *Actinobacillus succinogenes,* which includes a DNA molecule (i.e., a polynucleotide) comprising a transcription promoter for *Actinobacillus succinogenes* operationally linked to a heterologous *zwf or mdh* gene (e.g., a *A*. *succinogenes promoter* linked to a Zwf coding sequence and a *A*. *succinogenes promoter* linked to a Mdh coding sequence). The transcription promoter may include the *A. succinogenes* phosphoenolpyruvate *(PckA)* carboxykinase promoter or a variant thereof, whose sequence is disclosed in U.S. Patent No. 8,119,377. The heterologous *zwf* gene may encode *E. coli* Zwischenferment enzyme or a variant thereof. The heterologous *mdh* gene may encode *E. coli* Mdh enzyme or a variant thereof. Optionally, the *zwf gene* and/or the *mdh* gene may be optimized for expression in *Actinobacillus succinogenes.* The DNA molecule may be epigenetic (e.g., present on a plasmid). The DNA molecule may include a selectable marker (e.g., kanamycin resistance, ampicillin resistance, streptomycin resistance, sulfonamide resistance, tetracycline resistance, chloramphenicol resistance, or a combination thereof).

In some embodiments, the DNA molecule (polynucleotide) comprises a transcription promoter for a succinic acid producing bacteria operationally linked to a heterologous zwf gene. The transcription promoter may include a zwf promoter. The DNA molecule may be present within a plasmid. The DNA molecule may be present in a host cell (e.g., a host cell that produces succinic acid or propionic acid at concentrations of about 50 g/L to about 150 g/L).

In one embodiment, the method for producing succinic acid is provided. The method comprises fermenting a nutrient medium with a recombinant bacteria that expresses a heterologous *zwf* gene to produce a glucose-6-phosphate dehydrogenase enzymeand a heterologous *mdh* gene to produce a malate dehydrogenase enzyme. The recombinant bacteria may include a recombinant strain of *Actinobacillus succinogenes* (e.g., *A. succinogenes* recombinant strain deposited under ATCC Accession Number PTA-6255). The recombinant bacteria may include a recombinant strain of *Bisgaard Taxon 6, Bisgaard Taxon 10* and the like. The heterologous *zwf gene* may include the *E. coli zwf* gene. The heterologous *mdh* gene may include the *E. coli mdh* gene. Optionally, the recombinant strain is resistant to levels of sodium monofluoroacetate of at least about 1 g/L. Optionally, the recombinant strain is capable of producing succinic acid or propionic acid at concentrations of about 50 g/L to about 150 g/L. The nutrient medium may include a fermentable sugar (e.g., glucose). Typically, the method results in a succinic acid yield (g) of at least about 100% relative to glucose (g).

In another embodiment, the recombinant bacteria is a recombinant strain of a succinic acid producing bacteria. The recombinant bacteria comprises (e.g., has been transformed with) a heterologous *zwf* gene (i.e., a heterologous polynucleotide encoding a glucose-6-phosphate dehydrogenase). The heterologous *zwf* gene may be optimized for expression in the bacteria. In some embodiments, the heterologous *zwf* gene may encode E. *coli* Zwf enzyme.

In another embodiment, the recombinant bacteria is a recombinant strain of a succinic acid producing bacteria that has been transformed with a DNA molecule (a polynucleotide) that includes a transcription promoter for phosphoenolpyruvate *(PckA)* carboxykinase operationally linked to polynucleotide encoding a polypeptide having Zwf enzyme activity. The recombinant bacteria optionally has been transformed with a DNA molecule (a polynucleotide) that includes a transcription promoter for phosphoenolpyruvate *(PckA)* carboxykinase operationally linked to polynucleotide encoding a polypeptide having Mdh enzyme activity. The transcription promoter may include the *Actinobacillus succinogenes* phosphoenolpyruvate *(PckA)* carboxykinase promoter. In another embodiment, the recombinant bacteria is a recombinant strain transformed with a DNA molecule that is epigenetic. The DNA molecule may be present on a plasmid.

In another embodiment, the recombinant bacteria is a recombinant strain that is capable of producing succinic acid or propionic acid at concentrations of about 50 g/L to about 150 g/L.

The recombinant strain may be resistant to levels of sodium monofluoroacetate of at least about 1 g/L. In some embodiments, the recombinant strain is produced using recombinant *Actinobacillus succinogenes* deposited under ATCC Accession Number PTA-6255, or the recombinant strain is *Actinobacillus succinogenes* deposited under ATCC Accession Number PTA-120462.

In another embodiment, the recombinant bacteria is used for producing succinic acid or propionic acid in a method that includes fermenting a nutrient medium with the recombinant bacteria. The nutrient medium typically includes fermentable sugar such as glucose or sorbitol. The method may result in a succinic acid yield (g) of at least about 70%, at least about 80%, at least about 90%, or at least about 100% relative to glucose (g). The method may result in a propionic acid (g) of at least about 62% to about 72% relative to glucose (g), or at least about 60%, or at least about 70%, or at least about 80%, or at least about 90% (or any range within these percentages).

The methods for producing an organic acid can include growing suitable bacteria in a suitable fermentation broth which contains a carbon source (e.g., crude sorbitol). In one embodiment, the fermentation broth also contains a nitrogen source, inorganic salts, vitamins or growth promoting factors, and the like. In some embodiments, the salts, ammonium source and other nutrient media requirements may be obtained from corn steep liquor (CSL), a by-product of the corn wet-milling industry. With respect to salts, sodium sources include, but are not limited to, Na₃PO₄, Na₂HPO₄, NaH₂PO₄, Na₂CO₃, NaHCO₃, NaCl, and Na from organic salts (e.g. monosodium glutamate, sodium acetate). The sodium concentration can range between about 1200 mg/l to about 6800 mg/L. In some embodiments, the sodium concentration is from about 3000 mg/l to about 3500 mg/L. The invention is not limited to sodium salts; other salts are contemplated as part of the invention.

Fermentations can be conducted by combining the carbon source and fermentation broth in any suitable fermentor, and inoculating with a suitable bacteria. Fermentation may be carried out either aerobically or anaerobically under conditions conducive to the growth of the bacteria and production of the suitable organic acid. In one embodiment, fermentation temperature is maintained within the range of at least about 25° C, and less than about 50° C. In some embodiments, the temperature is between about 30°C and about 39° C (e.g., about 38° C). In some embodiments, the temperature is between about 30° C and about 37°C.

The fermentation broth may also include a betaine or an addition salt or a mixture thereof. In some embodiments the betaine is betaine-HCl, betaine free base or the like.

In other embodiments, the betaine may be present as a component of a feed product which contains betaine. Exemplary betaines or at least one feed product which contains betaine include betaine, amino acid fermentation byproduct solubles, molasses containing betaine, condensed separator byproduct, condensed molasses solubles, vinasse, or any mixture thereof. Other examples of feed products which contain betaine include a condensed, extracted glutamic acid fermentation product, amino acid fermentation byproduct solubles from the fermentative production lysine, amino acid fermentation byproduct solubles from the fermentative production threonine, or amino acid fermentation byproduct solubles from the fermentative production tryptophan. The betaine concentration may range from about 0.05 g/l to about 2 g/l. In some embodiments the betaine concentration used may be 0.2g/l to 0.5g/l.

In one embodiment, the pH of the fermentation broth at the beginning of fermentation is within the range of about pH 6-7 or the range of about pH 7 to about pH 8.0 (e.g., about pH 8.0). Fermentation pH can be controlled by addition of base, e.g., pH may be controlled to achieve about pH 5 to about pH 7, or about pH 6 to about pH 7 (e.g., about pH 6.6), or about pH 4.5 to about 6, or from about 5 to about 5.5, as the fermentation progresses. Magnesium bases are suitable for controlling pH in the context of the invention. In one embodiment, MgCO₃ is provided to control the pH in a CO₂ atmosphere to optionally achieve an approximate pH of about pH 6.0 to about pH 7.0 (e.g., about pH 6.4 to about pH 6.8), preferably about pH 6.6. Mg(OH)₂ also is appropriate. NH₄OH, NaOH, gaseous NH₃, Na₃PO₄ or their carbonate forms may be used, although the method is not dependent on a particular pH control agent.

### EXAMPLES

### Example 1

### Bacteria Strains and Plasmids

*A. succinogenes* strains FZ45 and FZ53 are stable bacterial variants of *Actinobacillus succinogenes* 130Z, which is resistant to sodium monofluoroacetate. See Guettler et al., INT'L J. SYST. BACT. (1999) 49:207-216; and U.S. Patent No. 5,573,931. An *E. coli-A. succinogenes* shuttle vector pLS88 (deposited at the American Type Culture Collection as ATCC accession no. 86980) was obtained from Dr. Leslie Slaney, University of Manitoba, Canada. Plasmid pLS88 is described as having been isolated from *Haemophilus ducreyi* and may confer resistance to sulfonamides, streptomycin, and kanamycin.

### Genetic Manipulations:

Recombinant DNA manipulations generally followed methods described in the art. Plasmid DNA was prepared by the alkali lysis method. Typical resuspension volumes for multicopy plasmids extracted from 1.5 ml cultures were 50 µl. Larger DNA preparation used the Qiagen Plasmid Purification Midi and Maxi kit according to the manufacturer's instructions. Restriction endonucleases, molecular weight standards, and pre-stained markers were purchased from New England Biolabs and Invitrogen and digests were performed as recommended by the manufacturers, except that an approximately 5-fold enzyme excess was used. DNA was analyzed on Tris-acetate-agarose gels in the presence of ethidium bromide. DNA was extracted from agarose gels and purified using the Qiagen gel extraction kit according to the manufacturer's instructions. DNA was dephosphorylated using shrimp or calf alkaline phosphatase (Roche) in combination with restriction digests. The phosphatase was heat inactivated at 70 °C. for 15 min or by passing through a Qiagen purification column. Ligations were performed using a 3- to 5-fold molar excess of insert to vector DNA in a 20 µl reaction volume and 1 µl of T4 DNA Ligase (New England Biolabs) for 1 hour at 25° C. *E. coli* transformation was performed by using "library efficiency competent cells" purchased from Invitrogen, following the manufacturer's instructions.

Transformations using ligation mixes were plated without dilutions on standard LB plates containing the appropriate antibiotic. PCR amplifications were carried out using the Perkin Elmer manual as a guideline. Primer designs were based on published sequences (as provided the National Center for Biotechnology Information (NCBI) database) and obtained from *Invitrogen Life Sciences.* The primers included engineered restriction enzyme recognition sites. Primers were analyzed for dimer and hairpin formation and melting temperature using the Vector NTI program. All primers were ordered from the Michigan State Macromolecular Structure Facility. PCR amplifications were carried out in an Eppendorf Gradient Master Cycler, or in a Perkin Elmer Thermocycler. Starting annealing temperatures were determined using the Vector NTI program for each primer pair. Restriction enzymes for digesting the amplified products were purchased from Invitrogen or New England Biolabs.

*A. succinogenes* competent cells for electroporation were prepared by growing cells in the presence of MgCO₃ in tryptic soy broth, TSB, medium supplemented with glucose and harvested in early to mid log-phase. Unused carbonate was removed by low speed centrifugation. Cells were spun down, washed twice with sterile water, twice with 10% v/v glycerol, and resuspended in 0.01x the original culture volume of 10% glycerol. Cells were suspended into small aliquots, flash frozen and stored at -80°C. 40µl of prepared cells were used for electroporation, using 0.1cm cuvettes and a BioRad GenePulser with settings of 400W, 25mF, 1.8kV. Following electroporation, 1ml room temperature TSB medium was immediately added to the cuvette and incubated at 37 °C for 1 h. The cell solution was plated on TSB agar plates containing the appropriate antibiotic, and incubated for 3 days at 37°C in a CO₂ atmosphere.

### Plasmid p830.60

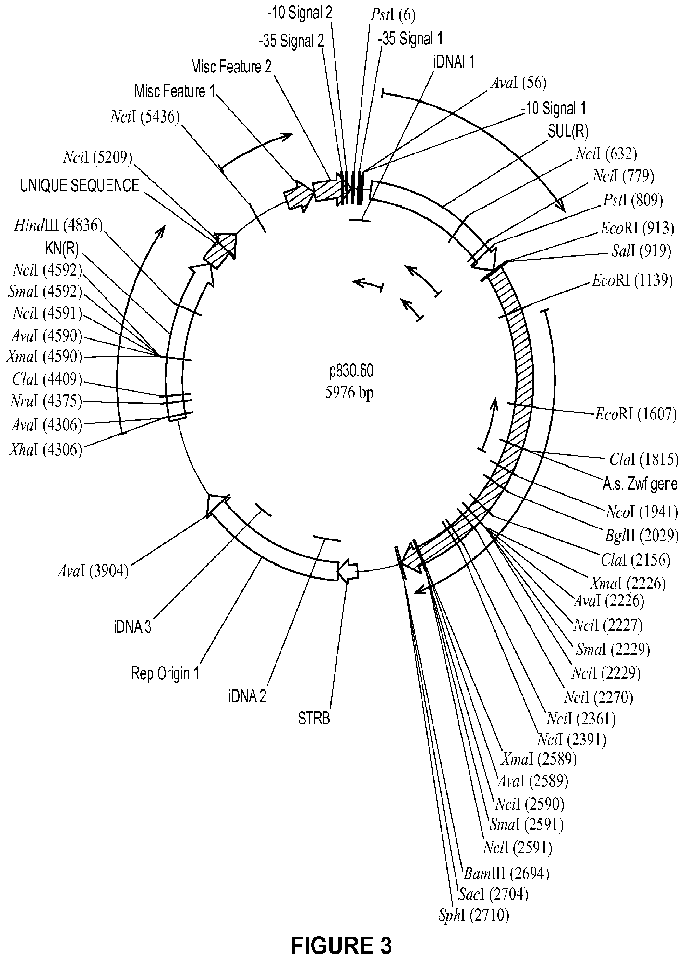

The *A. succinogenes zwf gene* and promoter were amplified from *Actinobacillus succinogenes* FZ45 genomic DNA, using primer TD299 (SEQ ID No. 5), and TD300, (SEQ ID No. 6), and inserted into the BamH1 and Sal1 sites of pJR762.47, a derivative of pLS88, with a multicloning site, inserted into the *EcoRI* and *SphI* sites of pLS88 and shown as Fig 3.

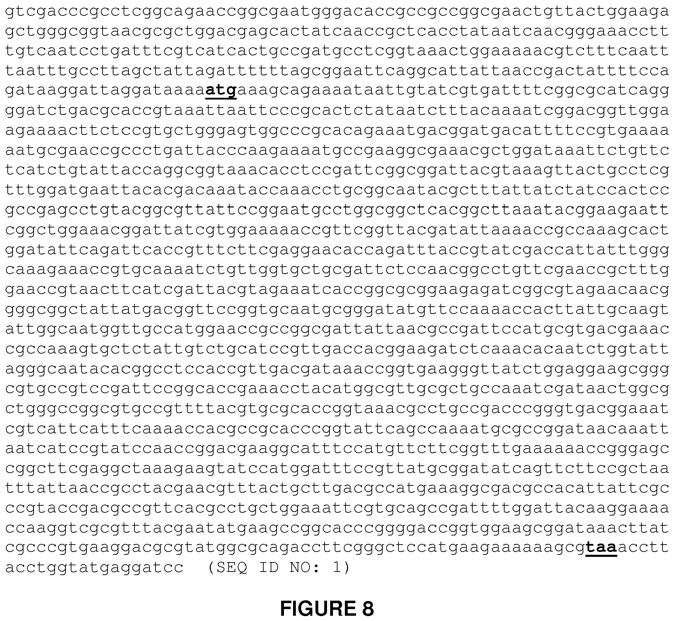

The cloned gene in p830.60 is 1781 base pairs (bp), the coding region starts at position 272 (bold underlined atg in Fig. 8) and stops at 1757 (bold underlined taa in Fig. 8) and its sequence is provided as SEQ ID NO:1. The promoter sequence begins at the start and ends before the coding sequence.

### Plasmid pISTONS1

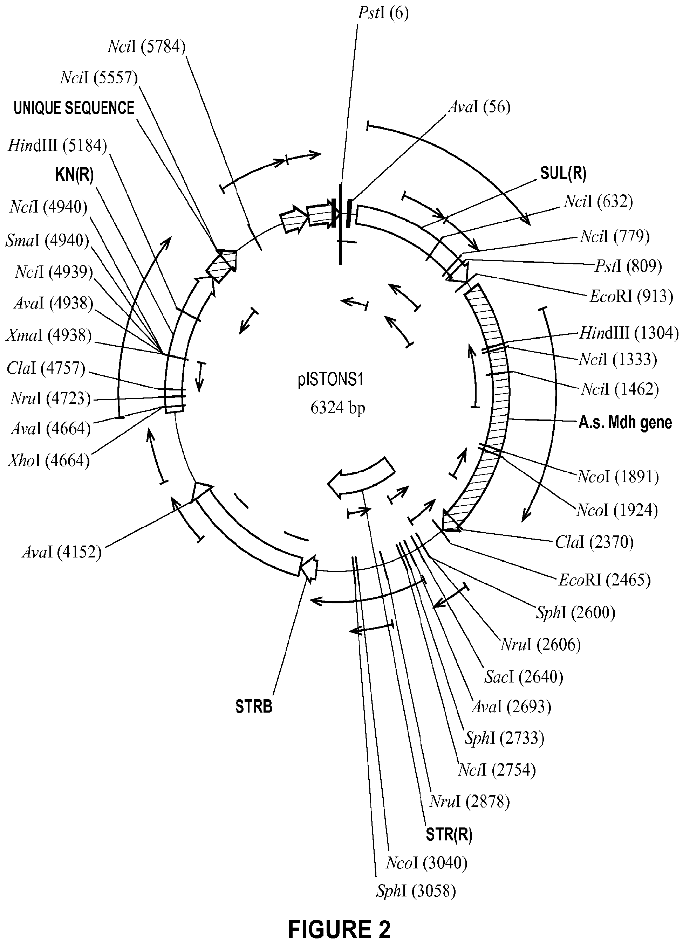

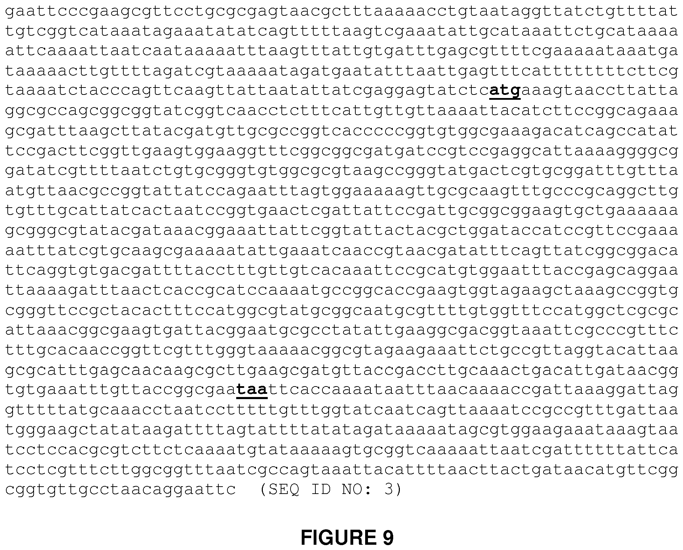

The *A. succinogenes* Mdh gene and promoter were amplified from *Actinobacillus succinogenes* FZ45 genomic DNA using primers TD223 (SEQ ID NO: 7), and TD218 (SEQ ID NO: 8). The amplified DNA was cloned as an EcoRI fragment and inserted into the EcoRI site of pLS88 as shown in Fig 2. The *mdh* gene in pISTONS1 is 1558 bp, with the coding region starting at 303 (bold underlined atg in Fig. 9) and ending at 1239 (bold underlined taa in Fig. 9) and provided as SEQ ID NO: 3 and the promoter is shown from the start of the sequence and ends before the coding region.

### Plasmid p856.78

The *A. succinogenes mdh* gene was excised from pISTONS1 using the restriction enzyme EcoRI, the ends were filled in with Klenow Polymerase to prepare blunt ends and ligated to plasmid p830.60, which had been linearized with the restriction endonuclease BamHI and the ends had been filled in with Klenow Polymerase. The two genes are arranged as head-to-tail insertions.

### Example 2

### Succinic Acid Production from Sorbitol by Overexpression of Zwf and Mdh Enzymes

*Actinobacillus succinogenes* strains (FZ53; FZ53/pLS88; FZ53/p830.60; FZ53/pPISTONS1 and FZ53/p856.78) were cultivated in fermentation vessels (Bioflo III, New Brunswick) containing 2 L of culture medium. The culture medium contained 120 g/L sorbitol, 30 g/L (solids), corn steep liquid (CSL) (10-12% solids from ADM), 1.6 g/L Mg(OH)₂, 0.2 mg/L biotin, 0.5g/L betaine HCl, 0.2 mg/L MSG, 6.5 mM sodium phosphate, 7 g/L Na2CO3, 0.5 g/L yeast extract (AG900).

The fermentor was inoculated with 6.25 % inoculum from a vial culture cultivated in the same medium as the fermentor (but omitting the Mg(OH)₂) and incubated with constant shaking at 150 rpm at 38 °C for 13 h.

Inoculated fermentors were incubated at 38 °C with agitation at 380 rev/min and a sparge of 0.025 to 0.05 vvm CO₂. The pH of the fermentation medium was maintained at pH 6.8 by automatic addition of 6 M Mg(OH)₂ or addition of MgCO₃ at 120 g/L.

A carbon source feed was implemented between 12 and 22 h during which 15 g of additional carbon source were added to the fermentation vessel.

### Sugar and organic acid determination:

Samples were removed from the fermentors at intervals and the solids removed by centrifugation at 10,000 x g for 4 min. The supernatant was filtered through a 0.2 µM filter prior to analysis. Sugar and organic acid concentrations in the culture supernatants and filtrates were determined by HPLC (Agilent 1200 series). An Aminex HPX-87H (300 mm x 7.8 mm) column (Bio-Rad) was used with a mobile phase consisting of 0.013 N H₂SO₄ with a flow rate of 1.4 ml/min. Analyte peaks were detected and quantified using a refractive index detector (Waters 2414), identification of peaks was determined by reference to organic acid standard solutions purchased from Sigma. Table 1 below shows the fermentation evaluation of multiple *A. succinogenes* transformants, grown on sorbitol.

**Table: 1**

| | | Fermentation Performance | | | |
|---|---|---|---|---|---|
| Strain designation | Construct | Titer (g/L) | Productivity (g/L-h | Yield (g succinic acid/g sugar) | n= |
| FZ53 | No vector | 109.23 ± 2.50 | 2.83 ± 0.12 | 0.99 ± 0.02 | 3 |
| FZ53 PLS88 | Empty vector | 107.00 ±2.01 | 2.77 ± 0.06 | 0.97 ± 0.01 | 3 |
| FZ53 p830.60 | zwf | 113.08 0.85 | 2.70 ± 0.17 | 1.03 ± 0.01 | 3 |
| FZ53 pPISTONS1 | mdh | 120.53 ± 0.33 | 2.20 ± 0.14 | 1.10 ± 0.01 | 2 |
| FZ53 p856.78 | zwf;mdh | 120.72 ± 1.98 | 2.50 ± 0.11 | 1.12 ± 0.02 | 6 |

The transformation of *A. succinogenes* with an "empty" vector (pLS88) that contained no genes to be overexpressed by the host did not significantly impact the performance of the succinic acid fermentation when sorbitol was the carbon source. Over expression of either the *zwf* gene or the *mdh* gene as single genes (vectors p830.60 and pPISTONS1 respectively) increased the succinic acid yield. The highest yield was obtained with the overexpression of Mdh enzyme, however this high yield came at the expense of the productivity, which was lower in this transformant than in the Zwf expressing strain, the untransformed parent and the transformant with the empty vector.

The co-expression of *zwf* and *mdh* genes also lead to a high yield (equivalent to that observed for the mdh transformant), but also supported an increased productivity (not significantly different from the *zwf* transformant.

It is demonstrated that the co-expression of Zwf and Mdh enzymes promotes the formation of succinic acid with high productivity, yield and titer when sorbitol is the carbon source.

### Example 3

### Succinic Acid Production from Glucose by Overexpression of Zwf and Mdh Enzymes

*Actinobacillus succinogenes* strains (FZ53; FZ53/pLS88; FZ53/p830.60; FZ53/pISTONS1 and FZ53/p856.78) described in Example 1 were cultivated in 50 mL anaerobic vials in the same medium as described in Example 2 with the modification that the bottles were preloaded with 6 g MgCO₃ to maintain culture pH. The vials were inoculated with 2.5 mL (5 % v/v inoculum) from an identical seed vial. Vials were incubated at 38 °C with constant shaking at 150 rev/min.

Analysis of culture filtrates was carried out as described in Example 2. An example of the analysis results are as follows (calculated at 46 hours): FZ53 pLS88 achieved a titer of 80.0 g/L, a productivity of 1.74 g/L-h, and yield of 0.76 g succinic acid/g sugar; FZ53 p830.60 (Zwf) achieved a titer of 100.6 g/L, a productivity of 2.19 g/L-h, and yield of 0.89 g succinic acid/g sugar; FZ53 pPISTONS1 (Mdh) achieved a titer of 80.2 g/L, a productivity of 1.87 g/L-h, and yield of 0.77 g succinic acid/g sugar; and FZ53 p878.56 (Zwf + Mdh) achieved a titer of 110.0 g/L, a productivity of 2.16 g/L-h, and yield of 0.96 g succinic acid/g sugar. See also Table 2, which summarizes the results of fermentation evaluation of A. succinogenes transformants on glucose.

**Table 2:**

| **Strain** n=3 | **construct** | **Improvements [%]** | | |
|---|---|---|---|---|
| | | titer | Productivity | yield |
| FZ53/pLS88 | empty vector | 100.0 | 100.0 | 100.0 |
| FZ53/p830.60 | Zwf | 110.7 | 111.2 | 107.9 |
| FZ53/pISTONS1 | Mdh | 102.6 | 102.8 | 100.0 |
| FZ53/p856.78 | Zwf + Mdh | 117.5 | 114.0 | 122.4 |

Whilst the over expression of Zwf was confirmed to be beneficial, increasing both productivity and yield of succinic acid from glucose, the over expression of Mdh alone did not exhibit any beneficial impact. The co-expression of Zwf and Mdh did provide a benefit over the expression of Zwf alone, increasing the yield whilst not significantly decreasing the succinic acid productivity. Additional observations are provided below.

Succinic acid production using *A. succinogenes* is an anaerobic production process. The organism is a natural succinic acid producer. Production is stimulated by supplementation of culture with either H2 or more reduced substrates (e.g., sorbitol or mannitol), suggesting that reducing power (NADPH) limited succinic acid production in A. *succinogenes.* With this in mind, Zwf (an enzyme that directly generates NADPH and diverts glucose into the pentose phosphate pathway or Entner-Doudoroff pathway) was overexpressed in *A. succinogenes.* This intervention increased succinic acid fermentation performance in terms of yield. Therefore, the diversion of glucose from the glycolytic pathway through the pentose phosphate pathway or Entner-Doudoroff pathway (and increasing the intracellular NADPH pool) benefited succinic acid production.

Biochemical studies of *A. succinogenes* and comparison with succinic acid producing *E. coli* demonstrated that the enzymes involved in the conversion of phosphoenol pyruvate to succinic acid (phosphoenol pyruvate decarboxylase, malate dehydrogenase, fumarase, and fumarate reductase) are highly expressed in *A. succinogenes,* suggesting a mechanism behind the prodigious ability of *A. succinogenes* to accumulate succinic acid. In particular, malate dehydrogenase activity was found to be very high in *A. succinogenes -* greater than 10 fold higher than in *E. coli* (2100 nmol/min/mg protei

n c.f. 160 nmol/min/mg protein (Van der Werf, Arch. Microbiol., 167, 332-342 (1997)). Thus, malate dehydrogenase activity was not expected to limit the production of succinic acid in *A. succinogenes.* Indeed, Mdh over-expression alone failed to significantly improve titer, productivity, or yield compared to a control bacteria lacking a heterologous Mdh encoding polynucleotide. See Table 2. This observation appeared to confirm the conclusions drawn after the biochemical study of *A. succinogenes,* that Mdh was not a rate limiting step in the production of succinic acid in *A. succinogenes* as a result of its naturally high endogenous activity.

On the basis of the biochemical survey and the data reported in Table 2 for Mdh overexpression alone, there would be no expectation of a benefit of the co-expression of a polynucleotide encoding glucose-6-phosphate dehydrogenase (*zwf*) and a polynucleotide encoding a malate dehydrogenase (*mdh*) in *A. succinogenes.*

However, during characterization of a Zwf overexpressing strain of *A. succinogenes,* a peak in the HPLC by-product profile was surprisingly observed that was associated with, and increased with, Zwf overexpression. The peak was determined to correspond to oxaloacetate, a very labile compound that easily decarboxylates, dimerizes, and converts to malate. This suggested that, unexpectedly, when Zwf was overexpressed in *A. succinogenes,* Mdh became a limiting enzyme in succinic acid production, resulting in an accumulation of its substrate (oxaloacetate). Without wishing to be bound by a particular theory, the build-up of oxaloacetate as a byproduct diverts carbon flow, decreasing the succinic acid yield of the fermentation. Thus, contrary to biochemical *in vitro* studies that demonstrated that Mdh activity is high in *A. succinogenes,* enzyme activity *in vivo* was unexpectedly limited. Overproduction of Mdh in combination with Zwf overcame the limitation, avoiding the accumulation of oxaloacetate and increasing the yield of succinic acid.

This example demonstrates that the co-expression of Zwf and Mdh provides a benefit over the expression of either single enzyme when glucose is used as a carbon source. Mdh was previously shown not to be a limiting activity in the production of succinic acid by *A. succinogenes.* It was unexpectedly discovered that over-expression of Mdh lead to an improved performance in succinic acid production by *A. succinogenes* when Zwf was also overexpressed.

Some additional non-limiting embodiments are provided below to further exemplify the present invention.

In one aspect of the invention, the recombinant bacteria overexpresses glucose-6-phosphate dehydrogenase and malate dehydrogenase enzymes. In some embodiments a single enzyme or both enzymes are heterologous to the organism. In various embodiments, expression of the heterologous polynucleotide or overexpression of the polynucleotide encoding the glucose-6-phosphate dehydrogenase and/or malate dehydrogenase results in a greater than two-fold, five-fold, ten-fold, 25-fold, 50-fold, 100-fold, or 250-fold, or 500-fold increase in enzyme activity as compared with enzyme activity observed in a parent (unmodified, matched) bacteria.

The inventive bacteria in various aspects produces more organic acid (or produces organic acid more quickly or more efficiently) than an otherwise similar bacteria that does not comprise the heterologous polynucleotide(s) or does not overexpress the polynucleotide(s). In various embodiments, the bacteria produces at least 2%, at least 3%, at least 5%, at least 6%, at least 7%, at least 10%, at least 12%, at least 15%, at least 17%, at least 20%, at least 25%, or at least 30% more organic acid than an otherwise similar bacteria that does not comprise the heterologous polynucleotide(s) or does not overexpress the polynucleotide(s) in the same time frame. Alternatively or in addition, the bacteria demonstrates a level of productivity that is at least 2%, at least 3%, at least 5%, at least 6%, at least 7%, at least 10%, at least 12%, at least 15%, at least 17%, at least 20%, at least 25%, or at least 30% better than an unmodified (parent) bacteria. Alternatively or in addition, the bacteria demonstrates an increase in yield of at least 2%, at least 3%, at least 5%, at least 6%, at least 7%, at least 10%, at least 12%, at least 15%, at least 17%, at least 20%, at least 25%, or at least 30% compared to an unmodified (parent) bacteria.

In other embodiments both enzymes are expressed from *A. succinogenes.* In still other embodiments both enzymes are expressed from *E.coli.* In still other embodiments glucose-6-phoshphate dehydrogenase is encoded from *E.coli* and malate dehydrogenase is encoded by *A. succinogenes.* The gene combinations may be reversed. In other embodiments, the bacteria expressing Zwf and Mdh include members of the *Pasteurellaceae* family. In still other embodiments, the bacteria include *Actinobacillus succinogenes, Bisgaard Taxon* 6 and *Bisgaard Taxon* 10 or bacterias that have more than 90% rRNA sequence identity to *Actinobacillus succinogenes.* The host cell expressing these genes may be *A. succinogenes* In one embodiment organic acids are succinic acid or propionic acid.

In some embodiments crude polyols may be used as the carbon source. In other embodiments, the fermentation broth or medium to grow the bacteria include corn steep liquor, betaine, sodium, magnesium ions and combinations thereof.

The disclosure of the expression of enzymes that are used in the pentose phosphate pathway (or Entner-Doudoroff pathway) and the reductive tri-carboxylic acid cycle results in improved organic acid production than a single enzyme alone.

. For example, although described primarily as a single recombinant organism that coexpresses *zwf* and *mdh* it can be envisaged that multiple organisms that each express *zwf* and *mdh* may be included in a single fermentation process to result in organic acid production. The invention includes variants or progeny of the bacteria described herein that retain the phenotypic characteristics of the recombinant bacteria. A substantially pure monoculture of the bacteria described herein (i.e., a culture comprising at least 80% or at least 90% of a desired bacteria) also is provided.

## Claims

1. A recombinant bacteria (i) expressing heterologous glucose-6-phosphate dehydrogenase or overexpressing glucose-6-phosphate dehydrogenase and (ii) expressing heterologous malate dehydrogenase or overexpressing malate dehydrogenase, wherein the bacteria is a natural succinic acid producing bacteria.

2. A recombinant bacteria comprising (i) a non-native or overexpressed polynucleotide encoding a glucose-6-phosphate dehydrogenase and (ii) a non-native or overexpressed polynucleotide encoding a malate dehydrogenase.

3. The recombinant bacteria of claim 1 or claim 2, comprising a 16S ribosomal RNA sequence with at least 90% identity to the 16S ribosomal RNA sequence of Actinobacillus succinogenes.

4. The recombinant bacteria of claim 1 or claim 2, which is Actinobacillus succinogenes, Bisgaard Taxon 6 or Bisgaard Taxon 10.

5. The recombinant bacteria of any one of claims 1-4, wherein the glucose-6-phosphate dehydrogenase or malate dehydrogenase are A. succinogenes enzymes.

6. The recombinant bacteria of any one of claims 1-4, wherein the glucose-6-phosphate dehydrogenase and malate dehydrogenase are A. succinogenes enzymes.

7. The recombinant bacteria of any one of claims 1-5, wherein the glucose-6-phosphate dehydrogenase or malate dehydrogenase are E. coli enzymes.

8. The recombinant bacteria of any one of claims 1-4, wherein the glucose-6-phosphate dehydrogenase and malate dehydrogenase are E. coli enzymes.

9. The recombinant bacteria of any one of claims 1-4, wherein the glucose-6-phosphate dehydrogenase comprises an amino acid sequence having at least about 40% sequence identity to the amino acid sequence set forth in SEQ ID NO: 2.

10. The recombinant bacteria of any one of claims 1-4, wherein the malate dehydrogenase comprises an amino acid sequence having at least about 60% sequence identity to the amino acid sequence set forth in SEQ ID NO: 4.

11. The recombinant bacteria of any one of claims 1-10, wherein the bacteria demonstrates improved organic acid production compared to a bacteria expressing glucose-6-phosphate dehydrogenase or malate dehydrogenase enzymes alone.

12. The recombinant bacteria of any one of claims 1-11, wherein the bacteria is capable of producing succinic acid at concentrations of about 50g/L to 150g/L.

13. A process for organic acid production, which process comprises culturing the recombinant bacteria of any one of claims 1-12 in a fermentation medium.

14. A method of producing organic acid comprising the step of:
culturing a recombinant bacteria expressing glucose-6-phosphate dehydrogenase and malate dehydrogenase enzymes with a carbon source under conditions favoring organic acid production, wherein the enzymes are not native to the bacteria or are overexpressed compared to an unmodified parent bacteria.

15. A method of producing organic acid comprising culturing the recombinant bacteria of any one of claims 1-12 with a carbon source under conditions favoring organic acid production.

16. The method of any one of claims 13-15, wherein the organic acid is succinic acid or propionic acid.

17. The method of claim 16, wherein the organic acid is succinic acid.

18. The method of any one of claims 14-17, wherein the carbon source is glucose or polyol.

## Patentansprüche

1. Rekombinante Bakterien (i), die heterologe Glucose-6-phosphat-Dehydrogenase exprimieren oder Glucose-6-phosphat-Dehydrogenase überexprimieren und (ii), die heterologe Malat-Dehydrogenase exprimieren oder Malat-Dehydrogenase überexprimieren, wobei die Bakterien natürliche Bernsteinsäure erzeugende Bakterien sind.

2. Rekombinante Bakterien, umfassend (i) ein nicht-natives oder überexprimiertes Polynukleotid, das für eine Glucose-6-phosphat-Dehydrogenase kodiert und (ii) ein nicht-natives oder überexprimiertes Polynukleotid, das für eine Malat-Dehydrogenase kodiert.

3. Rekombinante Bakterien nach Anspruch 1 oder Anspruch 2, umfassend eine 16S ribosomale RNA-Sequenz mit mindestens 90% Identität zu der 16S ribosomalen RNA-Sequenz von Actinobacillus succinogenes.

4. Rekombinante Bakterien nach Anspruch 1 oder Anspruch 2, die Actinobacillus succinogenes, Bisgaard Taxon 6 oder Bisgaard Taxon 10 sind.

5. Rekombinante Bakterien nach einem der Ansprüche 1-4, wobei die Glucose-6-phosphat-Dehydrogenase oder Malat-Dehydrogenase A. succinogenes-Enzyme sind.

6. Rekombinante Bakterien nach einem der Ansprüche 1-4, wobei die Glucose-6-phosphat-Dehydrogenase und Malat-Dehydrogenase A. succinogenes-Enzyme sind.

7. Rekombinante Bakterien nach einem der Ansprüche 1-5, wobei die Glucose-6-phosphat-Dehydrogenase oder Malat-Dehydrogenase E. coli-Enzyme sind.

8. Rekombinante Bakterien nach einem der Ansprüche 1-4, wobei die Glucose-6-phosphat-Dehydrogenase und Malat-Dehydrogenase E. coli-Enzyme sind.

9. Rekombinante Bakterien nach einem der Ansprüche 1-4, wobei die Glucose-6-phosphat-Dehydrogenase eine Aminosäuresequenz mit mindestens etwa 40% Sequenzidentität zu der in SEQ ID NR.: 2 angeführten Aminosäuresequenz umfasst.

10. Rekombinante Bakterien nach einem der Ansprüche 1-4, wobei die Malat-Dehydrogenase eine Aminosäuresequenz mit mindestens etwa 60% Sequenzidentität zu der in SEQ ID NR.: 4 angeführten Aminosäuresequenz umfasst.

11. Rekombinante Bakterien nach einem der Ansprüche 1-10, wobei die Bakterien verbesserte organische Säure-Herstellung zeigen, verglichen mit Bakterien, die nur die Enzyme Glucose-6-phosphat-Dehydrogenase oder Malat-Dehydrogenase exprimieren.

12. Rekombinante Bakterien nach einem der Ansprüche 1-11, wobei die Bakterien in der Lage sind, Bernsteinsäure bei Konzentrationen von etwa 50g/l bis 150g/l zu erzeugen.

13. Verfahren zur organischen Säure-Herstellung, wobei das Verfahren Züchten der rekombinanten Bakterien nach einem der Ansprüche 1-12 in einem Fermentationsmedium umfasst.

14. Verfahren zur Herstellung von organischer Säure, umfassend den Schritt:
Züchten von rekombinanten Bakterien, die Glucose-6-phosphat-Dehydrogenase- und Malat-Dehydrogenase-Enzyme exprimieren, mit einer Kohlenstoffquelle unter Bedingungen, die die Erzeugung organischer Säure begünstigen, wobei die Enzyme für die Bakterien nicht nativ sind oder überexprimiert werden, verglichen mit unmodifizierten Stamm-Bakterien.

15. Verfahren zur Herstellung von organischer Säure, umfassend Züchten der rekombinanten Bakterien nach einem der Ansprüche 1-12 mit einer Kohlenstoffquelle unter Bedingungen, die die Erzeugung organischer Säure begünstigen.

16. Verfahren nach einem der Ansprüche 13-15, wobei die organische Säure Bernsteinsäure oder Propionsäure ist.

17. Verfahren nach Anspruch 16, wobei die organische Säure Bernsteinsäure ist.

18. Verfahren nach einem der Ansprüche 14-17, wobei die Kohlenstoffquelle Glucose oder Polyol ist.

## Revendications

1. Bactérie recombinante (i) exprimant une glucose-6-phosphate déshydrogénase hétérologue ou surexprimant une glucose-6-phosphate déshydrogénase et (ii) exprimant une malate déshydrogénase hétérologue ou surexprimant une malate déshydrogénase, dans laquelle la bactérie est une bactérie produisant de l'acide succinique naturel.

2. Bactérie recombinante comprenant (i) un polynucléotide non natif ou surexprimé codant pour une glucose-6-phosphate déshydrogénase et (ii) un polynucléotide non natif ou surexprimé codant pour une malate déshydrogénase.

3. Bactérie recombinante selon la revendication 1 ou 2, comprenant une séquence d'ARN ribosomique 16S avec au moins 90% d'identité avec la séquence d'ARN ribosomique 16S d'Actinobacillus succinogenes.

4. Bactérie recombinante selon la revendication 1 ou 2, qui est Actinobacillus succinogenes, Bisgaard Taxon 6 ou Bisgaard Taxon 10.

5. Bactérie recombinante selon l'une quelconque des revendications 1 à 4, dans laquelle la glucose-6-phosphate déshydrogénase ou la malate déshydrogénase sont des enzymes A. succinogenes.

6. Bactérie recombinante selon l'une quelconque des revendications 1 à 4, dans laquelle la glucose-6-phosphate déshydrogénase et la malate déshydrogénase sont des enzymes A. succinogenes.

7. Bactérie recombinante selon l'une quelconque des revendications 1 à 5, dans laquelle la glucose-6-phosphate déshydrogénase ou la malate déshydrogénase sont des enzymes E. coli.

8. Bactérie recombinante selon l'une quelconque des revendications 1 à 4, dans laquelle la glucose-6-phosphate déshydrogénase et la malate déshydrogénase sont des enzymes E. coli.

9. Bactérie recombinante selon l'une quelconque des revendications 1 à 4, dans laquelle la glucose-6-phosphate déshydrogénase comprend une séquence d'acides aminés ayant au moins environ 40% d'identité de séquence avec la séquence d'acides aminés présentée dans SEQ ID NO : 2.

10. Bactérie recombinante selon l'une quelconque des revendications 1 à 4, dans laquelle la malate déshydrogénase comprend une séquence d'acides aminés ayant au moins environ 60% d'identité de séquence avec la séquence d'acides aminés présentée dans SEQ ID NO : 4.

11. Bactérie recombinante selon l'une quelconque des revendications 1 à 10, dans laquelle la bactérie démontre une production d'acide organique améliorée par rapport à une bactérie exprimant les enzymes glucose-6-phosphate déshydrogénase ou malate déshydrogénase seules.

12. Bactérie recombinante selon l'une quelconque des revendications 1 à 11, dans laquelle la bactérie est capable de produire de l'acide succinique aux concentrations d'environ 50 g/L à 150 g/L.

13. Procédé de production d'acide organique, lequel procédé comprend la culture de la bactérie recombinante selon l'une quelconque des revendications 1 à 12 dans un milieu de fermentation.

14. Procédé de production d'acide organique comprenant l'étape de :
culture d'une bactérie recombinante exprimant les enzymes glucose-6-phosphate déshydrogénase et malate déshydrogénase avec une source de carbone dans des conditions favorisant la production d'acide organique, dans lequel les enzymes ne sont pas natives de la bactérie ou sont surexprimées par rapport à une bactérie parente non modifiée.

15. Procédé de production d'acide organique comprenant la culture de la bactérie recombinante selon l'une quelconque des revendications 1 à 12 avec une source de carbone dans des conditions favorisant la production d'acide organique.

16. Procédé selon l'une quelconque des revendications 13 à 15, dans lequel l'acide organique est l'acide succinique ou propionique.

17. Procédé selon la revendication 16, dans lequel l'acide organique est l'acide succinique.

18. Procédé selon l'une quelconque des revendications 14 à 17, dans lequel la source de carbone est le glucose ou un polyol.


### SEQUENCE LISTING

<110> THE MICHIGAN BIOTECHNOLOGY INSTITUTE Guettler, Michael Jadhav, Sachin Kleff, Susanne Hanchar, Robert
<120> RECOMBINANT MICRORGANISMS FOR PRODUCING ORGANIC ACIDS
<130> 32236/40000A
<150> US-61/708,998
   <151> 2012-10-02
<160> 10
<170> PatentIn version 3.5
<210> 1
   <211> 1781
   <212> DNA
   <213> Actinobacillus succinogenes
<220>
   <221> misc_feature
   <223> Zwf gene
<400> 1
   gtcgacccgc ctcggcagaa ccggcgaatg ggacaccgcc gccggcgaac tgttactgga 60
   agagctgggc ggtaacgcgc tggacgagca ctatcaaccg ctcacctata atcaacggga 120
   aacctttgtc aatcctgatt tcgtcatcac tgccgatgcc tcggtaaact ggaaaaacgt 180
   ctttcaattt aatttgcctt agctattaga ttttttagcg gaattcaggc attattaacc 240
   gactattttc cagataagga ttaggataaa aatgaaagca gaaaataatt gtatcgtgat 300
   tttcggcgca tcaggggatc tgacgcaccg taaattaatt cccgcactct ataatcttta 360
   caaaatcgga cggttggaag aaaacttctc cgtgctggga gtggcccgca cagaaatgac 420
   ggatgacatt ttccgtgaaa aaatgcgaac cgccctgatt acccaagaaa atgccgaagg 480
   cgaaacgctg gataaattct gttctcatct gtattaccag gcggtaaaca cctccgattc 540
   ggcggattac gtaaagttac tgcctcgttt ggatgaatta cacgacaaat accaaacctg 600
   cggcaatacg ctttattatc tatccactcc gccgagcctg tacggcgtta ttccggaatg 660
   cctggcggct cacggcttaa atacggaaga attcggctgg aaacggatta tcgtggaaaa 720
   accgttcggt tacgatatta aaaccgccaa agcactggat attcagattc accgtttctt 780
   cgaggaacac cagatttacc gtatcgacca ttatttgggc aaagaaaccg tgcaaaatct 840
   gttggtgctg cgattctcca acggcctgtt cgaaccgctt tggaaccgta acttcatcga 900
   ttacgtagaa atcaccggcg cggaagagat cggcgtagaa caacggggcg gctattatga 960
   cggttccggt gcaatgcggg atatgttcca aaaccactta ttgcaagtat tggcaatggt 1020
   tgccatggaa ccgccggcga ttattaacgc cgattccatg cgtgacgaaa ccgccaaagt 1080
   gctctattgt ctgcatccgt tgaccacgga agatctcaaa cacaatctgg tattagggca 1140
   atacacggcc tccaccgttg acgataaacc ggtgaagggt tatctggagg aagcgggcgt 1200
   gccgtccgat tccggcaccg aaacctacat ggcgttgcgc tgccaaatcg ataactggcg 1260
   ctgggccggc gtgccgtttt acgtgcgcac cggtaaacgc ctgccgaccc gggtgacgga 1320
   aatcgtcatt catttcaaaa ccacgccgca cccggtattc agccaaaatg cgccggataa 1380
   caaattaatc atccgtatcc aaccggacga aggcatttcc atgttcttcg gtttgaaaaa 1440
   accgggagcc ggcttcgagg ctaaagaagt atccatggat ttccgttatg cggatatcag 1500
   ttcttccgct aatttattaa ccgcctacga acgtttactg cttgacgcca tgaaaggcga 1560
   cgccacatta ttcgcccgta ccgacgccgt tcacgcctgc tggaaattcg tgcagccgat 1620
   tttggattac aaggaaaacc aaggtcgcgt ttacgaatat gaagccggca cccggggacc 1680
   ggtggaagcg gataaactta tcgcccgtga aggacgcgta tggcgcagac cttcgggctc 1740
   catgaagaaa aaagcgtaaa ccttacctgg tatgaggatc c 1781
<210> 2
   <211> 495
   <212> PRT
   <213> Actinobacillus succinogenes
<220>
   <221> MISC_FEATURE
   <223> Zwf protein
<400> 2
   Met Lys Ala Glu Asn Asn Cys Ile Val Ile Phe Gly Ala Ser Gly Asp
   1               5                   10                  15
   Leu Thr His Arg Lys Leu Ile Pro Ala Leu Tyr Asn Leu Tyr Lys Ile
               20                  25                  30
   Gly Arg Leu Glu Glu Asn Phe Ser Val Leu Gly Val Ala Arg Thr Glu
           35                  40                  45
   Met Thr Asp Asp Ile Phe Arg Glu Lys Met Arg Thr Ala Leu Ile Thr
       50                  55                  60
   Gln Glu Asn Ala Glu Gly Glu Thr Leu Asp Lys Phe Cys Ser His Leu
   65                  70                  75                  80
   Tyr Tyr Gln Ala Val Asn Thr Ser Asp Ser Ala Asp Tyr Val Lys Leu
                   85                  90                  95
   Leu Pro Arg Leu Asp Glu Leu His Asp Lys Tyr Gln Thr Cys Gly Asn
               100                 105                 110
   Thr Leu Tyr Tyr Leu Ser Thr Pro Pro Ser Leu Tyr Gly Val Ile Pro
           115                 120                 125
   Glu Cys Leu Ala Ala His Gly Leu Asn Thr Glu Glu Phe Gly Trp Lys
       130                 135                 140
   Arg Ile Ile Val Glu Lys Pro Phe Gly Tyr Asp Ile Lys Thr Ala Lys
   145                 150                 155                 160
   Ala Leu Asp Ile Gln Ile His Arg Phe Phe Glu Glu His Gln Ile Tyr
                   165                 170                 175
   Arg Ile Asp His Tyr Leu Gly Lys Glu Thr Val Gln Asn Leu Leu Val
               180                 185                 190
   Leu Arg Phe Ser Asn Gly Leu Phe Glu Pro Leu Trp Asn Arg Asn Phe
           195                 200                 205
   Ile Asp Tyr Val Glu Ile Thr Gly Ala Glu Glu Ile Gly Val Glu Gln
       210                 215                 220
   Arg Gly Gly Tyr Tyr Asp Gly Ser Gly Ala Met Arg Asp Met Phe Gln
   225                 230                 235                 240
   Asn His Leu Leu Gln Val Leu Ala Met Val Ala Met Glu Pro Pro Ala
                   245                 250                 255
   Ile Ile Asn Ala Asp Ser Met Arg Asp Glu Thr Ala Lys Val Leu Tyr
               260                 265                 270
   Cys Leu His Pro Leu Thr Thr Glu Asp Leu Lys His Asn Leu Val Leu
           275                 280                 285
   Gly Gln Tyr Thr Ala Ser Thr Val Asp Asp Lys Pro Val Lys Gly Tyr
       290                 295                 300
   Leu Glu Glu Ala Gly Val Pro Ser Asp Ser Gly Thr Glu Thr Tyr Met
   305                 310                 315                 320
   Ala Leu Arg Cys Gln Ile Asp Asn Trp Arg Trp Ala Gly Val Pro Phe
                   325                 330                 335
   Tyr Val Arg Thr Gly Lys Arg Leu Pro Thr Arg Val Thr Glu Ile Val
               340                 345                 350
   Ile His Phe Lys Thr Thr Pro His Pro Val Phe Ser Gln Asn Ala Pro
   355 360 365
   Asp Asn Lys Leu Ile Ile Arg Ile Gln Pro Asp Glu Gly Ile Ser Met
   370 375 380
   Phe Phe Gly Leu Lys Lys Pro Gly Ala Gly Phe Glu Ala Lys Glu Val
   385 390 395 400
   Ser Met Asp Phe Arg Tyr Ala Asp Ile Ser Ser Ser Ala Asn Leu Leu
   405 410 415
   Thr Ala Tyr Glu Arg Leu Leu Leu Asp Ala Met Lys Gly Asp Ala Thr
   420 425 430
   Leu Phe Ala Arg Thr Asp Ala Val His Ala Cys Trp Lys Phe Val Gln
   435 440 445
   Pro Ile Leu Asp Tyr Lys Glu Asn Gln Gly Arg Val Tyr Glu Tyr Glu
   450 455 460
   Ala Gly Thr Arg Gly Pro Val Glu Ala Asp Lys Leu Ile Ala Arg Glu
   465 470 475 480
   Gly Arg Val Trp Arg Arg Pro Ser Gly Ser Met Lys Lys Lys Ala
   485 490 495
<210> 3
   <211> 1558
   <212> DNA
   <213> Actinobacillus succinogenes
<220>
   <221> misc_feature
   <223> mdh gene
<400> 3
   gaattcccga agcgttcctg cgcgagtaac gctttaaaaa cctgtaatag gttatctgtt 60
   ttattgtcgg tcataaatag aaatatatca gtttttaagt cgaaatattg cataaattct 120
   gcataaaaat tcaaaattaa tcaataaaaa tttaagttta ttgtgatttg agcgttttcg 180
   aaaaataaat gataaaaact tgttttagat cgtaaaaata gatgaatatt taattgagtt 240
   tcattttttt tcttcgtaaa atctacccag ttcaagttat taatattatc gaggagtatc 300
   tcatgaaagt aaccttatta ggcgccagcg gcggtatcgg tcaacctctt tcattgttgt 360
   taaaattaca tcttccggca gaaagcgatt taagcttata cgatgttgcg ccggtcaccc 420
   ccggtgtggc gaaagacatc agccatattc cgacttcggt tgaagtggaa ggtttcggcg      480
   gcgatgatcc gtccgaggca ttaaaagggg cggatatcgt tttaatctgt gcgggtgtgg      540
   cgcgtaagcc gggtatgact cgtgcggatt tgtttaatgt taacgccggt attatccaga      600
   atttagtgga aaaagttgcg caagtttgcc cgcaggcttg tgtttgcatt atcactaatc      660
   cggtgaactc gattattccg attgcggcgg aagtgctgaa aaaagcgggc gtatacgata      720
   aacggaaatt attcggtatt actacgctgg ataccatccg ttccgaaaaa tttatcgtgc      780
   aagcgaaaaa tattgaaatc aaccgtaacg atatttcagt tatcggcgga cattcaggtg      840
   tgacgatttt acctttgttg tcacaaattc cgcatgtgga atttaccgag caggaattaa      900
   aagatttaac tcaccgcatc caaaatgccg gcaccgaagt ggtagaagct aaagccggtg      960
   cgggttccgc tacactttcc atggcgtatg cggcaatgcg ttttgtggtt tccatggctc     1020
   gcgcattaaa cggcgaagtg attacggaat gcgcctatat tgaaggcgac ggtaaattcg     1080
   cccgtttctt tgcacaaccg gttcgtttgg gtaaaaacgg cgtagaagaa attctgccgt     1140
   taggtacatt aagcgcattt gagcaacaag cgcttgaagc gatgttaccg accttgcaaa     1200
   ctgacattga taacggtgtg aaatttgtta ccggcgaata attcaccaaa ataatttaac     1260
   aaaaccgatt aaaggattag gtttttatgc aaacctaatc ctttttgttt ggtatcaatc     1320
   agttaaaatc cgccgtttga ttaatgggaa gctatataag attttagtat tttatataga     1380
   taaaaatagc gtggaagaaa taaagtaatc ctccacgcgt cttctcaaaa tgtataaaaa     1440
   gtgcggtcaa aaattaatcg atttttattt catcctcgtt tcttggcggt ttaatcgcca     1500
   gtaaattaca ttttaactta ctgataacat gttcggcggt gttgcctaac aggaattc       1558
<210> 4
   <211> 312
   <212> PRT
   <213> Actinobacillus succinogenes
<220>
   <221> MISC_FEATURE
   <223> mdh protein
<400> 4
   Met Lys Val Thr Leu Leu Gly Ala Ser Gly Gly Ile Gly Gln Pro Leu
   1               5                   10                  15
   Ser Leu Leu Leu Lys Leu His Leu Pro Ala Glu Ser Asp Leu Ser Leu
               20                  25                  30
   Tyr Asp Val Ala Pro Val Thr Pro Gly Val Ala Lys Asp Ile Ser His
           35                  40                  45
   Ile Pro Thr Ser Val Glu Val Glu Gly Phe Gly Gly Asp Asp Pro Ser
       50                  55                  60
   Glu Ala Leu Lys Gly Ala Asp Ile Val Leu Ile Cys Ala Gly Val Ala
   65                  70                  75                  80
   Arg Lys Pro Gly Met Thr Arg Ala Asp Leu Phe Asn Val Asn Ala Gly
                   85                  90                  95
   Ile Ile Gln Asn Leu Val Glu Lys Val Ala Gln Val Cys Pro Gln Ala
               100                 105                 110
   Cys Val Cys Ile Ile Thr Asn Pro Val Asn Ser Ile Ile Pro Ile Ala
           115                 120                 125
   Ala Glu Val Leu Lys Lys Ala Gly Val Tyr Asp Lys Arg Lys Leu Phe
       130                 135                 140
   Gly Ile Thr Thr Leu Asp Thr Ile Arg Ser Glu Lys Phe Ile Val Gln
   145                 150                 155                 160
   Ala Lys Asn Ile Glu Ile Asn Arg Asn Asp Ile Ser Val Ile Gly Gly
                   165                 170                 175
   His Ser Gly Val Thr Ile Leu Pro Leu Leu Ser Gln Ile Pro His Val
               180                 185                 190
   Glu Phe Thr Glu Gln Glu Leu Lys Asp Leu Thr His Arg Ile Gln Asn
           195                 200                 205
   Ala Gly Thr Glu Val Val Glu Ala Lys Ala Gly Ala Gly Ser Ala Thr
       210                 215                 220
   Leu Ser Met Ala Tyr Ala Ala Met Arg Phe Val Val Ser Met Ala Arg
   225                 230                 235                 240
   Ala Leu Asn Gly Glu Val Ile Thr Glu Cys Ala Tyr Ile Glu Gly Asp
                   245                 250                 255
   Gly Lys Phe Ala Arg Phe Phe Ala Gln Pro Val Arg Leu Gly Lys Asn
               260                 265                 270
   Gly Val Glu Glu Ile Leu Pro Leu Gly Thr Leu Ser Ala Phe Glu Gln
           275                 280                 285
   Gln Ala Leu Glu Ala Met Leu Pro Thr Leu Gln Thr Asp Ile Asp Asn
       290                 295                 300
   Gly Val Lys Phe Val Thr Gly Glu
   305                 310
<210> 5
   <211> 29
   <212> DNA
   <213> Artificial sequences
<220>
   <223> Artificial Zwf primer
<400> 5
   aaaggatcct cataccaggt aaggtttac 29
<210> 6
   <211> 29
   <212> DNA
   <213> Artificial sequences
<220>
   <223> Artificial Zwf primer
<400> 6
   aaagtcgacc cgcctcggca gaaccggcg 29
<210> 7
   <211> 28
   <212> DNA
   <213> Artificial sequences
<220>
   <223> Artificial Mdh primer
<400> 7
   ccgaattccc gaagcgttcc tgcgcgag 28
<210> 8
   <211> 27
   <212> DNA
   <213> Artificial sequences
<220>
   <223> Artificial Mdh primer
<400> 8
   aagaattcct gttaggcaac accgccg 27
<210> 9
   <211> 491
   <212> PRT
   <213> Escherichia coli
<300>
   <308> UniProtKB/Swiss-Prot / POAC53.1
   <309> 2013-09-18
   <313> (1)..(491)
<400> 9
   Met Ala Val Thr Gln Thr Ala Gln Ala Cys Asp Leu Val Ile Phe Gly
   1               5                   10                  15
   Ala Lys Gly Asp Leu Ala Arg Arg Lys Leu Leu Pro Ser Leu Tyr Gln
               20                  25                  30
   Leu Glu Lys Ala Gly Gln Leu Asn Pro Asp Thr Arg Ile Ile Gly Val
           35                  40                  45
   Gly Arg Ala Asp Trp Asp Lys Ala Ala Tyr Thr Lys Val Val Arg Glu
       50                  55                  60
   Ala Leu Glu Thr Phe Met Lys Glu Thr Ile Asp Glu Gly Leu Trp Asp
   65                  70                  75                  80
   Thr Leu Ser Ala Arg Leu Asp Phe Cys Asn Leu Asp Val Asn Asp Thr
                   85                  90                  95
   Ala Ala Phe Ser Arg Leu Gly Ala Met Leu Asp Gln Lys Asn Arg Ile
               100                 105                 110
   Thr Ile Asn Tyr Phe Ala Met Pro Pro Ser Thr Phe Gly Ala Ile Cys
           115                 120                 125
   Lys Gly Leu Gly Glu Ala Lys Leu Asn Ala Lys Pro Ala Arg Val Val
       130                 135                 140
   Met Glu Lys Pro Leu Gly Thr Ser Leu Ala Thr Ser Gln Glu Ile Asn
   145                 150                 155                 160
   Asp Gln Val Gly Glu Tyr Phe Glu Glu Cys Gln Val Tyr Arg Ile Asp
                   165                 170                 175
   His Tyr Leu Gly Lys Glu Thr Val Leu Asn Leu Leu Ala Leu Arg Phe
               180                 185                 190
   Ala Asn Ser Leu Phe Val Asn Asn Trp Asp Asn Arg Thr Ile Asp His
           195                 200                 205
   Val Glu Ile Thr Val Ala Glu Glu Val Gly Ile Glu Gly Arg Trp Gly
       210                 215                 220
   Tyr Phe Asp Lys Ala Gly Gln Met Arg Asp Met Ile Gln Asn His Leu
   225                 230                 235                 240
   Leu Gln Ile Leu Cys Met Ile Ala Met Ser Pro Pro Ser Asp Leu Ser
                   245                 250                 255
   Ala Asp Ser Ile Arg Asp Glu Lys Val Lys Val Leu Lys Ser Leu Arg
               260                 265                 270
   Arg Ile Asp Arg Ser Asn Val Arg Glu Lys Thr Val Arg Gly Gln Tyr
           275                 280                 285
   Thr Ala Gly Phe Ala Gln Gly Lys Lys Val Pro Gly Tyr Leu Glu Glu
       290                 295                 300
   Glu Gly Ala Asn Lys Ser Ser Asn Thr Glu Thr Phe Val Ala Ile Arg
   305                 310                 315                 320
   Val Asp Ile Asp Asn Trp Arg Trp Ala Gly Val Pro Phe Tyr Leu Arg
                   325                 330                 335
   Thr Gly Lys Arg Leu Pro Thr Lys Cys Ser Glu Val Val Val Tyr Phe
               340                 345                 350
   Lys Thr Pro Glu Leu Asn Leu Phe Lys Glu Ser Trp Gln Asp Leu Pro
           355                 360                 365
   Gln Asn Lys Leu Thr Ile Arg Leu Gln Pro Asp Glu Gly Val Asp Ile
       370                 375                 380
   Gln Val Leu Asn Lys Val Pro Gly Leu Asp His Lys His Asn Leu Gln
   385                 390                 395                 400
   Ile Thr Lys Leu Asp Leu Ser Tyr Ser Glu Thr Phe Asn Gln Thr His
                   405                 410                 415
   Leu Ala Asp Ala Tyr Glu Arg Leu Leu Leu Glu Thr Met Arg Gly Ile
               420                 425                 430
   Gln Ala Leu Phe Val Arg Arg Asp Glu Val Glu Glu Ala Trp Lys Trp
           435                 440                 445
   Val Asp Ser Ile Thr Glu Ala Trp Ala Met Asp Asn Asp Ala Pro Lys
       450                 455                 460
   Pro Tyr Gln Ala Gly Thr Trp Gly Pro Val Ala Ser Val Ala Met Ile
   465                 470                 475                 480
   Thr Arg Asp Gly Arg Ser Trp Asn Glu Phe Glu
                   485                 490
<210> 10
   <211> 312
   <212> PRT
   <213> Escherichia coli
<300>
   <308> UniProtKB/Swiss-Prot / P61889.1
   <309> 2013-05-29
   <313> (1)..(312)
<400> 10
   Met Lys Val Ala Val Leu Gly Ala Ala Gly Gly Ile Gly Gln Ala Leu
   1               5                   10                  15
   Ala Leu Leu Leu Lys Thr Gln Leu Pro Ser Gly Ser Glu Leu Ser Leu
               20                  25                  30
   Tyr Asp Ile Ala Pro Val Thr Pro Gly Val Ala Val Asp Leu Ser His
           35                  40                  45
   Ile Pro Thr Ala Val Lys Ile Lys Gly Phe Ser Gly Glu Asp Ala Thr
       50                  55                  60
   Pro Ala Leu Glu Gly Ala Asp Val Val Leu Ile Ser Ala Gly Val Ala
   65                  70                  75                  80
   Arg Lys Pro Gly Met Asp Arg Ser Asp Leu Phe Asn Val Asn Ala Gly
                   85                  90                  95
   Ile Val Lys Asn Leu Val Gln Gln Val Ala Lys Thr Cys Pro Lys Ala
               100                 105                 110
   Cys Ile Gly Ile Ile Thr Asn Pro Val Asn Thr Thr Val Ala Ile Ala
           115                 120                 125
   Ala Glu Val Leu Lys Lys Ala Gly Val Tyr Asp Lys Asn Lys Leu Phe
       130                 135                 140
   Gly Val Thr Thr Leu Asp Ile Ile Arg Ser Asn Thr Phe Val Ala Glu
   145                 150                 155                 160
   Leu Lys Gly Lys Gln Pro Gly Glu Val Glu Val Pro Val Ile Gly Gly
                   165                 170                 175
   His Ser Gly Val Thr Ile Leu Pro Leu Leu Ser Gln Val Pro Gly Val
               180                 185                 190
   Ser Phe Thr Glu Gln Glu Val Ala Asp Leu Thr Lys Arg Ile Gln Asn
           195                 200                 205
   Ala Gly Thr Glu Val Val Glu Ala Lys Ala Gly Gly Gly Ser Ala Thr
       210                 215                 220
   Leu Ser Met Gly Gln Ala Ala Ala Arg Phe Gly Leu Ser Leu Val Arg
   225                 230                 235                 240
   Ala Leu Gln Gly Glu Gln Gly Val Val Glu Cys Ala Tyr Val Glu Gly
                   245                 250                 255
   Asp Gly Gln Tyr Ala Arg Phe Phe Ser Gln Pro Leu Leu Leu Gly Lys
               260                 265                 270
   Asn Gly Val Glu Glu Arg Lys Ser Ile Gly Thr Leu Ser Ala Phe Glu
           275                 280                 285
   Gln Asn Ala Leu Glu Gly Met Leu Asp Thr Leu Lys Lys Asp Ile Ala
       290                 295                 300
   Leu Gly Glu Glu Phe Val Asn Lys
   305                 310